(19) Europäisches Patentamt
European Patent Office
Office européen des brevets

(11) EP 4 741 394 A1

(12) EUROPEAN PATENT APPLICATION
published in accordance with Art. 153(4) EPC

(43) Date of publication:
13.05.2026 Bulletin 2026/20

(21) Application number: 23944009.2

(22) Date of filing: 04.07.2023

(51) International Patent Classification (IPC):
*C07D 487/04* (2006.01) *C07D 471/04* (2006.01)
*A61K 31/5025* (2006.01) *A61K 31/437* (2006.01)
*A61P 35/00* (2006.01)

(52) Cooperative Patent Classification (CPC):
**A61K 31/437; A61K 31/5025; A61P 35/00; C07D 471/04; C07D 487/04**

(86) International application number:
**PCT/CN2023/105714**

(87) International publication number:
**WO 2025/007275 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Beijing Pearl Biotechnology Limited Liability Company**
  **Beijing 100102 (CN)**
- **Beijing Avistone Biotechnology Co., Ltd.**
  **Beijing 100102 (CN)**

(72) Inventors:
- **SONG, Weizhou**
  **Beijing 100163 (CN)**
- **LI, Gang**
  **Beijing 100163 (CN)**
- **ZHANG, Peilong**
  **Beijing 100163 (CN)**
- **SHI, Hepeng**
  **Beijing 100163 (CN)**

(74) Representative: **IK-IP LTD**
**8 Devonshire Square**
**London EC2M 4YJ (GB)**

# (54) CRYSTAL FORM OF [1,2,4]TRIAZOLO[4,3-B]PYRIDAZINE COMPOUND, AND PREPARATION METHOD THEREFOR AND USE THEREOF

(57) A crystal form of a [1,2,4]triazolo[4,3-b]pyridazine compound, and a preparation method therefor and a use thereof. The crystal form is a crystal form of a free base and can be used for treating tumor diseases, and a crystal form B has the characteristic of storage stability. Additionally, the crystal form B has a relatively low incipient melting temperature, and when the crystal form B is used in a hot-melt extrusion process, the content of impurities of a related substance in a prepared sampled is low, thereby facilitating improvement of the quality of drugs.



Processed by Luminess, 75001 PARIS (FR)

## Description

### TECHNICAL FIELD

The present invention relates to the field of chemical medicine, and in particular to crystal form of a [1,2,4]triazolo [4,3-b]pyridazine compound as c-Met inhibitor, and preparation method therefor and use thereof.

### BACKGROUND

Hepatocyte growth factor receptor (c-Met) is a transmembrane protein encoded by the MET gene and belongs to the tyrosine kinase receptor superfamily, with the predominant expression in epithelial cells. Hepatocyte growth factor (HGF), also known as scatter factor (SF), is currently the main ligand for c-Met with a high ligand/receptor affinity. It is widely present in various human tissues and organs and is mainly expressed in interstitial cells. When HGF binds to c-Met in an extracellular domain, it triggers c-Met dimerization and promotes the transphosphorylation of two catalytic active sites Tyr1234 and Tyr1235 in a c-Met activation loop, which in turn leads to autophosphorylation of Tyr1349 and Tyr1356 in the C terminus of the receptor protein, thereby recruiting a variety of downstream cellular effectors and effector molecules, activating a series of downstream signaling pathways, such as PI3K-Akt, Ras-MAPK, STAT and Wnt/β-catenin etc. c-Met/HGF is crucial for promoting cell proliferation, cell growth, cell migration, vascular invasion and angiogenesis, c-Met gene abnormalities mainly include three types, namely: MET exon 14 skipping mutation, MET gene amplification and c-Met protein overexpression, c-Met gene abnormalities may cause abnormal activation of the c-Met pathway, leading to overactivation of downstream pathways to induce cancer.

At present, c-Met small-molecule kinase inhibitors are the most prevalent and therapeutically promising method for blocking the c-Met/HGF signaling pathway . These small-molecule kinase inhibitors can act on the catalytic domain within the membrane to prevent protein phosphorylation, thereby blocking signal transduction and achieving targeted cancer treatment.

6-(1-Cyclopropyl-1-hydro-pyrazol-4-yl)-3-(difluoro(6-fluoro-2-methyl-(dihydro-indazol-5-yl)methyl)-[1,2,4]triazolo[4,3-b]pyridazine is a highly selective c-Met inhibitor for treating cancers caused by c-Met gene abnormalities. It has been granted breakthrough therapy designation by China National Medical Products Administration (NMPA) for the treatment of nonsmall cell lung cancer patients with c-Met exon 14 mutations. Its structural formula is represented by formula (I) as follows:

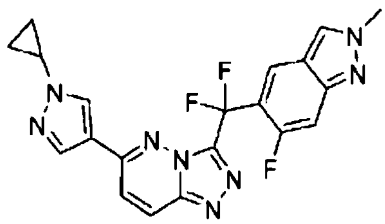

(I)

At present, there are no published reports on the crystal forms of the free base of the compound represented by formula (I).

### SUMMARY OF THE INVENTION

In order to overcome the defects of the prior art, the present invention provides crystal form of a [1,2,4]triazolo [4,3-b]pyridazine compound as a c-Met inhibitor, and preparation method therefor and use thereof. The crystal forms of the present invention are free base and used for treating tumor diseases. The crystal forms of the present invention have good chemical and physical stability and low hygroscopicity, and are less affected by heat, humidity, and light, thereby facilitating storage. Most importantly, a crystal form B of the present invention has a relatively low incipient melting temperature, and when the crystal form B is used in a hot-melt extrusion process, a sample preparation process is smooth, and a prepared solid dispersion has a low impurity content of related substance, which is beneficial to improving the quality of pharmaceuticals.

The present invention provides a crystal form A of 6-(1-cyclopropyl-1-hydro-pyrazol-4-yl)-3-(difluoro(6-fluoro-2-methyl-(dihydro-indazol-5-yl)methyl)-[1,2,4]triazolo[4,3-b]pyridazine represented by formula (I), wherein with Cu-Kα radiation, the crystal form A has characteristic peaks at 2θvalues of 7.47°, 10.30°, 12.47°, 14.18°, 17.19°, 24.25°, and 25.43° in a X-ray powder diffraction pattern expressed in 2θ angles, with a 2θ error range of ± 0.2°,

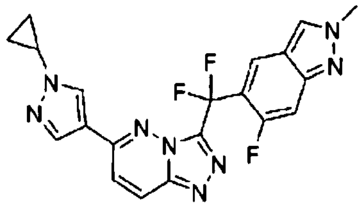

(I)

In some embodiments of the present invention, the X-ray powder diffraction pattern of the crystal form A shows characteristic peaks at 2θ values of 7.47°, 10.30°, 12.47°, 14.18°, 17.19°, 17.54°, 17.85°, 18.15°, 20.39°, 24.25°, 25.43°, 25.97°, and 26.43°, with a 2θ error range of ± 0.2°.

In some embodiments of the present invention, the X-ray powder diffraction pattern of the crystal form A is shown in FIG. 1.

In some embodiments of the present invention, the X-ray powder diffraction pattern of the crystal form A shows characteristic peaks having 2θ values shown in the table below, with a 2θ error range of ± 0.2°.

| No. | 2θ angle (°) | No. | 2θ angle (°) | No. | 2θ angle (°) | No. | 2θ angle (°) |
|---|---|---|---|---|---|---|---|
| 1 | 7.47° | 12 | 17.54° | 23 | 22.90° | 34 | 28.57° |
| 2 | 8.20° | 13 | 17.85° | 24 | 23.18° | 35 | 29.27° |
| 3 | 10.30° | 14 | 18.15° | 25 | 23.76° | 36 | 29.75° |
| 4 | 11.26° | 15 | 18.86° | 26 | 24.25° | 37 | 31.93° |
| 5 | 12.47° | 16 | 19.31° | 27 | 24.75° | 38 | 33.91° |
| 6 | 13.79° | 17 | 19.75 | 28 | 25.43° | 39 | 35.50° |
| 7 | 14.18° | 18 | 20.10° | 29 | 25.97° | 40 | 36.94° |
| 8 | 14.89° | 19 | 20.39° | 30 | 26.43° | 41 | 37.46° |
| 9 | 15.86° | 20 | 20.98° | 31 | 27.08° | 42 | 38.36° |
| 10 | 16.36° | 21 | 21.97° | 32 | 27.53° | / | / |
| 11 | 17.19° | 22 | 22.48° | 33 | 28.01° | / | / |

In some embodiments of the present invention, a thermogravimetric analysis (TGA) curve of the crystal form A shows no weight loss from room temperature to a melting point.

In some embodiments of the present invention, a TGA thermogram of the crystal form A is shown in FIG. 2.

In some embodiments of the present invention, a differential scanning calorimetry (DSC) curve of the crystal form A shows an endothermic peak value at 222.27 °C.

In some embodiments of the present invention, a DSC thermogram of the crystal form A is shown in FIG. 3.

The present invention provides a preparation method for the crystal form A defined above. The preparation method comprises: dissolving the compound of formula (I) in a normal solvent A1 at 0 °C to 70 °C, filtering, adding the resulting clear filtrate dropwise to an anti-solvent A2 to obtain the crystal form A; and
preferably, the normal solvent A1 is acetic acid.

Preferably, the anti-solvent A2 is ethanol.

Preferably, a volume ratio of the normal solvent A1 to the anti-solvent A2 is 1/6 to 1/1, more preferably 1/4 or 1/3.

Preferably, a temperature at which the compound of formula (I) is dissolved in the normal solvent A1 is 20 °C to 40 °C, more preferably 35 °C, to enable clear dissolution of the compound of formula (I) in the normal solvent A1.

Or, the preparation method comprises: dissolving the compound of formula (I) in a normal solvent A1, filtering, and adding an anti-solvent A2 dropwise to the resulting clear filtrate to obtain the crystal form A; and
preferably, the normal solvent A1 is acetic acid.

Preferably, the anti-solvent A2 is ethanol.

Preferably, a volume ratio of the normal solvent A1 to the anti-solvent A2 is 1/6 to 1/1, more preferably 1/4 or 1/3.

Preferably, a temperature at which the compound of formula (I) is dissolved in the normal solvent A1 is 20 °C to 50 °C, more preferably 35 °C.

Or, the preparation method comprises: dissolving the compound of formula (I) in a certain volume of solvent or mixed solvent, heating until complete dissolution, and filtering and slow cooling to obtain the crystal form A; and

preferably, a warming temperature is 20 °C to 70 °C, more preferably 50 °C.

Preferably, the solvent is acetonitrile, methanol or acetic acid.

Preferably, the mixed solvent is a mixture of acetic acid and ethanol.

Preferably, a volume ratio of the mixed solvent is 1/6 to 1/1, more preferably 1/4 or 1/3.

The present invention further provides a crystal form B of 6-(1-cyclopropyl-1-hydro-pyrazol-4-yl)-3-(difluoro(6-fluoro-2-methyl-(dihydro-indazol-5-yl)methyl)-[1,2,4]triazolo[4,3-b]pyridazine represented by formula (I), wherein with Cu-Kα radiation, the crystal form B has characteristic peaks at 2θvalues of 8.38°, 15.99°, 18.73°, 24.86°, 28.96°, and 29.83° in a X-ray powder diffraction pattern expressed in 2θ angles, with a 20 error range of ± 0.2°.

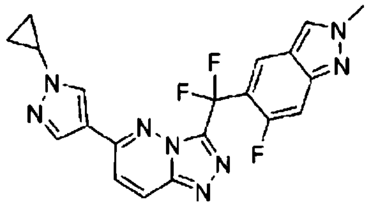

(I)

In some embodiments of the present invention, the X-ray powder diffraction pattern of the crystal form B shows characteristic peaks at 2θ values of 8.38°, 15.57°, 15.99°, 18.73°, 24.86°, 25.30°, 28.96°, and 29.83°, with a 2θ error range of ± 0.2°.

In some embodiments of the present invention, the X-ray powder diffraction pattern of the crystal form B is shown in FIG. 5.

In some embodiments of the present invention, the X-ray powder diffraction pattern of the crystal form B shows characteristic peaks having 20 values shown in the table below, with a 2θ error range of ± 0.2°.

| No. | 2θ angle (°) | No. | 2θ angle (°) | No. | 2θ angle (°) | No. | 2θ angle (°) |
|---|---|---|---|---|---|---|---|
| 1 | 8.38° | 4 | 16.69° | 7 | 23.14° | 10 | 27.01° |
| 2 | 15.57° | 5 | 17.68° | 8 | 24.86° | 11 | 28.96° |
| 3 | 15.99° | 6 | 18.73° | 9 | 25.30° | 12 | 29.83° |

In some embodiments of the present invention, a thermogravimetric analysis (TGA) thermogram of the crystal form B shows a weight loss of up to 0.50% from room temperature to 200 °C.

In some embodiments of the present invention, a TGA thermogram of the crystal form B is shown in FIG. 6.

In some embodiments of the present invention, a differential scanning calorimetry (DSC) thermogram of the crystal form B shows an endothermic peak value at 203.00 °C.

In some embodiments of the present invention, a DSC thermogram of the crystal form B is shown in FIG. 7.

In some embodiments of the present invention, the crystal form B is granular.

In some embodiments of the present invention, the crystal form B has a particle size D90 of 50 μm to 150 μm.

The present invention further provides a preparation method for the crystal form B defined above. The method comprises: adding the compound of formula (I) to a mixture of a normal solvent B1 and an anti-solvent B2, heating until dissolution, filtering and cooling, cultivating crystals, adding the anti-solvent B2, and cooling to 0 °C to obtain the crystal form B.

Preferably, the normal solvent B1 is acetone.

Preferably, the anti-solvent B2 is water.

Preferably, a heating temperature is 0 °C to 60 °C, preferably 60 °C.

Preferably, before cultivating the crystals, a cooling temperature is 40 °C to 0 °C, preferably 30 °C.

Preferably, in the mixture of the normal solvent B1 and the anti-solvent B2, a volume ratio of the normal solvent B1 to the anti-solvent B2 is 4/1 to 1/1, preferably 3/1. Or,

the preparation method comprises: adding the compound of formula (I) to a mixture of a normal solvent B1 and an anti-solvent B2, heating until dissolution, filtering and cooling to obtain the crystal form B.

Preferably, the normal solvent B1 is acetone.

Preferably, the anti-solvent B2 is water.

Preferably, a heating temperature is 0 °C to 60 °C, preferably 60 °C.

Preferably, a cooling temperature is 0 °C to 30 °C, preferably 0 °C to 10 °C.

Preferably, in the mixture of the normal solvent B1 and the anti-solvent B2, a volume ratio of the normal solvent B1 to the anti-solvent B2 is 3/1 to 4/1, preferably 4/1.

The present invention further provides a pharmaceutical preparation or a pharmaceutical composition, which comprises one or two of the crystal forms A and B.

According to the present invention, the pharmaceutical preparation or the pharmaceutical composition may further comprise a pharmaceutically acceptable carrier and/or vehicle.

Optionally, the pharmaceutical preparation or the pharmaceutical composition is a tablet, capsule, pill, granule, powder, suppository, injection, solution, suspension, ointment, patch, lotion, drop, liniment, or spray.

The present invention further provides a use of the crystal form A or B defined above or the pharmaceutical preparation or the pharmaceutical composition defined above in preparation of a medicament for treating c-Met abnormality-mediated diseases.

In some embodiments of the present invention, the c-Met abnormality-mediated diseases are tumor diseases; and

preferably, the tumor diseases comprise: head and neck cancer, nasopharyngeal cancer, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin lymphoma, stomach cancer, lung cancer, liver cancer, gastrointestinal stromal tumor, thyroid cancer, squamous cell carcinoma, cholangiocarcinoma, endometrial cancer, multiple myeloma or mesothelioma, atherosclerosis, or pulmonary fibrosis.

The present invention further provides a method for treating c-Met abnormality-mediated diseases. The method comprises: administering the crystal form B or A defined above or the pharmaceutical preparation or the pharmaceutical composition therefore; wherein,

preferably, the c-Met abnormality-mediated diseases are tumor diseases; and

preferably, the tumor diseases comprise: head and neck cancer, nasopharyngeal cancer, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin lymphoma, stomach cancer, lung cancer, liver cancer, gastrointestinal stromal tumor, thyroid cancer, squamous cell carcinoma, cholangiocarcinoma, endometrial cancer, multiple myeloma or mesothelioma, atherosclerosis, or pulmonary fibrosis.

## Technical Effects

The crystal forms in the present application have good chemical and physical stability and low hygroscopicity, and are less affected by heat, moisture and light, showing the characteristic of storage stability. In addition, the crystal form B of the present invention has a relatively low incipient melting temperature, and when the crystal form B is used in a hot-melt extrusion process, a prepared sample has a low impurity content of related substance, which is beneficial to the quality of pharmaceuticals. Moreover, the crystal form B is granular, which is more conducive to uniform mixing with excipients, resulting in reduced friction during mixing and higher smoothness during preparation of the solid dispersion.

## Definition and Description

Unless otherwise stated, the following terms and phrases as used herein are intended to have the following meanings.

"Chemical stability" refers to the extent to which the crystal forms provided by the present invention undergo degradation reactions under certain temperature, humidity, and light conditions.

"Physical stability" refers to the extent to which the crystal forms provided by the present invention undergo solid form transformation under high temperature, high humidity, and grinding conditions.

"Normal solvent" refers to the solvent in which the compound of formula (I) of the present application has good solubility, for example, one or more of acetic acid, tetrahydrofuran, acetone, acetonitrile, dichloromethane, methanol, dimethyl sulfoxide, and dimethylformamide; and "anti-solvent" refers to the solvent in which the compound of formula (I) of the present application has poor solubility, for example, one or more of water, n-hexane, pentane, methyl tert-butyl ether, toluene, ethyl acetate, butyl acetate, ethanol, n-propanol, and 2-methyltetrahydrofuran.

"Pharmaceutical composition" refers to a mixture of the crystal form(s) of the compound of the present invention and other chemical ingredients, for example, pharmaceutically acceptable carriers, excipients or diluents. The purpose of the pharmaceutical composition is to facilitate the drug administration process. The pharmaceutical composition may include pharmaceutically acceptable excipients such as pH regulators, buffering agents and toxicity regulators, for example, sodium acetate, sodium chloride, potassium chloride, calcium chloride, sodium lactate, etc., to simulate physiological conditions.

"Pharmaceutically acceptable carrier" refers to a pharmaceutically acceptable substance, ingredient or medium,

such as a liquid or solid filler, diluent, vehicle, solvent or potting material, which participates in loading or delivery of an active compound involved in the present invention from one location, body fluid, tissue, organ (internal or external), or body part to another location, body fluid, organ (internal or external), or body part. A pharmaceutically acceptable carrier may be a medium, a diluent, a vehicle or other materials that have no excessive toxicity or side effects and can be used in contact with animal tissues.

Some pharmaceutically acceptable carriers include: (1) saccharides, such as lactose, glucose and sucrose; (2) starches, such as corn starch and potato starch; (3) cellulose and its derivatives, such as sodium carboxymethyl cellulose, ethyl cellulose, and cellulose acetate; (4) tragacanth gum powder; (5) maltose; (6) gelatin; (7) talc; (8) vehicles, such as cocoa butter and suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol, and polyethylene glycol; (12) esters, such as ethyl oleate and ethyl laurate; (13) agar gel; (14) buffering agents, such as magnesium hydroxide and aluminum hydroxide; (15) alginic acid; (16) sterile pyrogen-free water; (17) normal saline; (18) Ringer's solution; (19) alcohols, such as ethanol and propanol; (20) phosphate buffer; and (21) other substances that are non-toxic and compatible with the dosage form, such as acetone.

Each pharmaceutically acceptable carrier should be compatible with other ingredients. For example, it should form a formulation with the compound provided in the present invention without causing excessive toxicity, irritation, allergic reaction, immunogenicity or other problems or complications to living biological tissues or organs, showing a reasonable benefit-risk ratio.

The pharmaceutical ingredients can be formulated into any suitable dosage form, such as solid dosage forms (e.g. tablets, capsules, powders, granules, etc.) and liquid dosage forms (e.g., aqueous solutions, emulsions, elixirs, syrups, etc.). Methods and techniques for preparing pharmaceutical compositions are well known, and the pharmaceutical compositions can be prepared according to conventional techniques, such as those provided in Remington, The Science and Practice of Pharmacy (Gennaro ed. 20th edition, Williams & Wilkins PA, USA) (2000).

## BRIEF DESCRIPTION OF THE DRAWINGS

FIG. 1 shows the XRPD form of a crystal form A of the compound of formula (I);

FIG. 2 shows the TGA thermogram of the crystal form A of the compound of formula (I);

FIG. 3 shows the DSC thermogram of the crystal form A of the compound of formula (I);

FIG. 4 shows the overlay of XRPD forms of solid samples prepared with preparation methods 1 and 2 for the crystal form A of the compound of formula (I);

FIG. 5 shows the XRPD form of a crystal form B of the compound of formula (I);

FIG. 6 shows the TGA thermogram of the crystal form B of the compound of formula (I);

FIG. 7 shows the DSC thermogram of the crystal form B of the compound of formula (I);

FIG. 8 shows the polarized light microscopy (PLM) images of the crystal form A obtained by reverse solventing-out crystallization of the compound of formula (I) at 30 °C, with image A showing the status of initial crystallization, image B showing the status following half dropwise addition, image C showing the status following completion of the dropwise addition, image D showing the status following incubation for 1 h, image E showing the status following incubation for 3 h, and image F showing the status before filtration;

FIG. 9 shows the PLM images of the crystal form A obtained by reverse solventing-out crystallization of the compound of formula (I) at 20 °C; with image A showing the status of initial crystallization, image B showing the status following half dropwise addition, and image C showing the status following completion of the dropwise addition;

FIG. 10 shows the PLM images of the crystal form B of the compound of formula (I), with image A showing the status of initial crystallization, image B showing the status following completion of crystal cultivation, image C showing the status following dropwise addition of half the water, image D showing the status following completion of water addition, image E showing the status following completion of incubation, and image F showing the status before filtration;

FIG. 11 shows the overlay of XRPD forms of the crystal form B of the compound of formula (I) during vacuum drying at 40 °C at various time points;

FIG. 12 shows the overlay of XRPD forms of the crystal form B of the compound of formula (I) during vacuum drying at 60 °C at various time points;

FIG. 13 shows the overlay of XRPD forms of the crystal form B of the compound of formula (I) drying at room temperature at various time points under the protection of nitrogen;

FIG. 14 shows the overlay of XRPD forms of the crystal form B of the compound of formula (I) drying at 40 °C at various time points under the protection of nitrogen;

FIG. 15 shows the overlay of XRPD forms of samples of the crystal form A of the compound of formula (I) at various test conditions;

FIG. 16 shows the overlay of XRPD forms of samples of the crystal form B of the compound of formula (I) at various test conditions;

FIG. 17 shows the overlay of XRPD forms of solids resulting from keeping the crystal form A of the compound of formula (I) in various media for 24 h;

FIG. 18 shows the overlay of XRPD forms of solids resulting from keeping the crystal form B of the compound of formula (I) in various media for 24 h.

## DETAILED DESCRIPTION OF THE INVENTION

The present invention is further illustrated below in combination with embodiments. These embodiments are intended to help explain the content of the present invention, instead of limiting the scope of the present invention.

Instruments and Analysis Methods

1. XRPD: X-ray powder diffraction

The crystal form of the samples was analyzed using a Bruker D8 ADVANCE or Bruker D8 Focus X-ray powder diffractometer. The samples were scanned with the 2θ scanning angle of 3° to 42°, the scanning step of 0.02°, and the scanning time of 0.05 s or 0.1 s or 0.2 s per step. The voltage and current for fluorescent tubes were 40 kV and 40 mA, respectively. During sample preparation, an appropriate amount of samples were placed on a sample tray and flattened with a glass sheet or other tools to ensure a smooth and flat surface.

2. TGA: thermogravimetric analysis

The samples were analyzed using TA Instruments Discovery TGA Q500. The samples were placed into an aluminum tray with the tare removed, automatically weighed by the system, and then heated a specified temperature at a rate of 10 °C/min under the protection of nitrogen.

3. DSC: differential scanning calorimetry

The samples were analyzed using TA Instruments Discovery DSC 25. 2-10 mg of the samples were weighed and placed into an aluminum tray, and heated a specified temperature at a rate of 10 °C/min under the protection of nitrogen (50 ml/min).

4. PLM: polarized light microscopy

The samples were analyzed using LEICA DM750P. A small amount of the samples were placed on a glass slide, dispersed with a drop of cedar oil, covered with a cover slip, and placed on a microscope slide stage, followed by adjustment to an appropriate magnification to obtain a sample image.

5. HPLC: high-performance liquid chromatography

Solubility and stability tests were carried out using Agilent 1260 HPLC under the following conditions: column: ZORBAX Eclipse Plus Cl8 4.6* 100 mm 3.5-Micron; flow rate: 1 mL/min; column temperature: 35 °C; injection volume: 10 μL; mobile phases: mobile phase A: 0.02 mol/L potassium dihydrogen phosphate, and mobile phase B: ACN; running time: 40 min; detector: UV at 210 nm; diluent: ACN; and injection concentration: 0.2 mg/mL. The elution procedures are shown in the table below.

| | Time (min) | A (%) | B (%) |
|---|---|---|---|
| Elution procedure | 0 | 90 | 10 |
| | 30 | 30 | 70 |
| | 31 | 90 | 10 |
| | 40 | 90 | 10 |

6. Particle size D90

The particle size was measured using a dry method. With a Malvern 3000 laser particle size analyzer equipped with an Aero S sample injector, an air compressor with the air pressure of greater than 6 bar, and a funnel slit of 1.0 mm to 1.5 mm, about 200 mg of the sample was placed into the funnel of the Areo S sample injector and then into a dispersion unit, and a start button was pressed to start the sample test.

Each sample was tested three times, and the average D90 (μm) of the three tests was reported. When D90 ≥ 10 μm, the relative standard deviation RSD% (n=3) should be ≤ 15%; and when D90 <10 μm, the relative standard deviation RSD% (n=3) should be ≤ 30%.

Example **1:** Preparation of Crystal Form A of Compound of formula (I)

1. Preparation method 1:

At 35 °C, 3 g of the compound of formula (I) (a crude product prepared according to the synthesis method of Example 44 of CN103122000A) was dissolved in 7.8 mL (2.6 vol) of acetic acid in a reactor 1 and maintain the temperature. At 35 °C, 31.2 mL (12 vol) of ethanol was added into a reactor 2 and maintain the temperature. A saturated solution from the reactor 1 was slowly added to the reactor 2; after completion of dropwise addition, the mixture was incubated at 30 °C for 3 h and filtered; and a filter cake was rinsed with ethanol and dried at 40 °C. The obtained solids were characterized by XRPD, TGA and DSC, as shown as in FIGS. 1, 2 and 3.

As can be seen from FIG. 1, the X-ray powder diffraction pattern of the crystal form A shows characteristic peaks at 2θ values of 7.47°, 10.30°, 12.47°, 14.18°, 17.19°, 17.54°, 17.85°, 18.15°, 20.39°, 24.25°, 25.43°, 25.97°, and 26.43°. The 2θ error range is ± 0.2°. As can be seen from FIG. 2, the TGA thermogram shows that the sample have no weight loss before melting. As can be seen from FIG. 3, the DSC thermogram shows that the melting point peak has the initiate melting temperature of 221.16 °C, the peak temperature of 222.27 °C, and the heat enthalpy value of 156.10 J/g.

Combining the TGA thermogram results in FIG. 2 and the DSC thermogram results in FIG. 3, it indicates that the crystal form A is a non-solvated crystal with no hygroscopicity and high stability.

2. Preparation method 2:

At 20 °C, 2 g of the compound of formula (I) (a crude product prepared according to the synthesis method of Example 44 of CN103122000A) was dissolved in 8 mL (4 vol) of acetic acid in a reactor 1 and maintain the temperature. At 35 °C, 24 mL (12 vol) of ethanol was added to the reactor 2 and maintain the temperature. A saturated solution from the reactor 1 was added to the reactor 2; crystal cultivation was performed for 30 minutes, followed by another dropwise addition; after completion of the dropwise addition, the mixture was incubated at 35 °C for 3 h, cooled to 0 °C for overnight, and then filtered; and a filter cake was rinsed with ethanol and dried at 40 °C. The obtained solids were characterized by the XRPD, as shown in FIG. 4.

As can be seen from FIG. 4, the overlay of the XRPD form of the solid obtained by the preparation method 2 and the XRPD form of the crystal form A of the preparation method 1 indicates that the crystal form obtained by the preparation method 2 is the same as that obtained by the preparation method 1, and both are crystal forms A.

Example 2: Preparation and Stability Investigation of Crystal Form B of Compound of formula (I)

At room temperature, 12 g of the compound of formula (I) (a crude product prepared according to the synthesis method of Example 44 of CN103122000A) and 156 mL of an acetone/water mixed solvent (3/1 (v/v), 13 vol) were added to a reactor, then heated to 60 °C, and the mixture became clear. The mixed was cooled to 35 °C at 1 °C/min, followed by a very small amount of solids observed in the system; when the temperature dropped to 30 °C, crystal cultivation was started and lasted for 1 h; and then 78 mL (6.5 vol) of water was added over 2.5 hours. After completion of the dropwise addition, the mixture was incubated at 30 °C for 3 h, then cooled to 0 °C at 10 °C/h, incubated overnight, and filtered to obtain light yellow solids. The solids were dried at 40 °C to obtain white crystalline powder. The obtained solids were characterized by XRPD, TGA and DSC, as shown in FIGS. 5, 6 and 7.

As can be seen from FIG. 5, the X-ray powder diffraction form of the crystal form B shows characteristic peaks at 2θ values of 8.38°, 15.57°, 15.99°, 18.73°, 24.86°, 25.30°, 28.96°, and 29.83°, with a 2θ error range of ± 0.2°. As can be seen from FIG. 6, the TGA thermogram shows that the sample undergoes gradual weight loss of 0.50% from room temperature to 200 °C, indicating the absence of crystallization water and crystallization solvent in the sample. As can be seen from FIG. 7, the DSC thermogram shows that the sample has an endothermic peak starting from 201.23 °C, with a peak temperature of 203.00 °C and an enthalpy value of 13.626 J/g, and this endothermic peak is caused by the melting of the sample; the sample has an exothermic peak starting from 205.31 °C, with a peak temperature of 206.67 °C and an enthalpy value of 7.6334. J/g, and this exothermic peak is caused by the exothermic crystal transformation of the sample; and there is an endothermic peak starting from 223.03 °C, with a peak temperature of 224.15 °C and an enthalpy value of 94.995 J/g, and this endothermic peak is caused by the endothermic melting of the sample.

The TGA thermogram results in FIG. 6 and the DSC thermogram results in FIG. 7 indicate the absence of crystallization water or crystallization solvent in the sample.

Example **3:** Investigation of Crystal Habits of Crystal Forms A and B of Compound of formula (I)

1. Investigation of the crystal habit of crystal form A of the compound of formula (I): the crystal form A was prepared according to the preparation method 1 of Example 1, and samples were taken for PLM after dropwise addition of the saturated solution for 5 minutes, after dropwise addition of half of the saturated solution, after completion of dropwise addition of the saturated solution, after incubation at 30 °C for 1 h, after incubation at 30 °C for 3 h, and before filtration, respectively, as shown in FIG. 8.
As can be seen from the PLM images in FIG. 8, crystals grow uniformly during the dropwise addition, with final products in the form of short rods. The particle size distribution of the products during the experiment and before filtration is uniform, and the particle size D90 of the final products is between 20 μm and 60 μm.

2. Investigation of the crystal habit of crystal form A of the compound of formula (I): the crystal form A was prepared according to the preparation method 2 of Example 1, and samples were taken for PLM after dropwise addition of the saturated solution for 4 min, after dropwise addition of half of the saturated solution, and after completion of dropwise addition of the saturated solution, as shown in FIG. 9.
As can be seen from the PLM images in FIG. 9, crystallization occurs spontaneously during reverse dropwise addition, with a large number of solids in the system and uniform crystal growth during the dropwise addition. The final products are in the form of short rods with a uniform particle size distribution, with the particle size D90 between 20 μm and 50 μm.

3. Investigation of the crystal habit of crystal form B of the compound of formula (I): the crystal form B was prepared according to the method of Example 2, and samples were taken for PLM after crystal cultivation at 30 °C for 15 min, after crystal cultivation at 30 °C for 1 h, after dropwise addition of half of the water, after completion of dropwise addition of the water, after incubation at 30 °C for 3 h, and before filtration, respectively, as shown in FIG. 10.
As can be seen from the PLM images in FIG. 10, crystals grow uniformly during the dropwise addition, with final products in the form of granules. The particle size distribution of the products during the experiment and before filtration is uniform, and the particle size D90 of the final products is between 50 μm and 150 μm.

Example **4:** Investigation on Impact of Drying Conditions on Stability of Crystal Form B of Compound of formula (I)

The crystal form B was prepared according to the method of Example 2; four wet samples each of 3 g were placed into four 40 × 25 mm watch glasses, respectively, and then dried at 40 °C or 60 °C under vacuum, under nitrogen atmosphere at room temperature or 40 °C; and samples were taken at 0 h, 8 h, 12 h or 24 h, 48 h for XRPD characterization,

as shown in FIGS. 11 to 14.

As can be seen from FIGS. 11 to 14, except the crystal form B, no other crystal forms appear during drying. and this crystal form demonstrates stability during drying.

Example **5:** Evaluation of Thermal and Moisture Stability of Crystal Forms A and B

A certain amount of samples of the crystal forms A (prepared according to the preparation method 1 of Example 1) and B (prepared according to the method of Example 2) of the compound of formula (I) were placed into a stability test chamber, and taken out for HPLC and XRPD testing after a certain period of time.

Test conditions were as follows: under open conditions at 25 °C with 60% RH (25 °C/60% RH) or at 40 °C with 75% RH (40 °C/75% RH), HPLC and XRPD was characterized after one week, two weeks, 1 month and 2 months respectively; and HPLC and XRPD were characterized under closed conditions at 80 °C for one day and under closed conditions with lighting for 10 days, respectively. The test results are shown in Table 1 and FIGS. 15 and 16.

Table 1: Results of Stability of Crystal Forms A and B

| Conditions | Time | Purity (%) | | Degradation (%) | | XRPD | |
|---|---|---|---|---|---|---|---|
| | | Crystal form A | Crystal form B | Crystal form A | Crystal form B | Crystal form A | Crystal form B |
| Start | | 99.98 | 99.98 | | | | |
| 25 °C/60% RH | 1 week | 99.98 | 99.98 | 0 | 0 | Unchanged | Unchanged |
| | 2 weeks | 99.98 | 99.98 | 0 | 0 | Unchanged | Unchanged |
| | 1 month | 99.98 | 99.98 | 0 | 0 | Unchanged | Unchanged |
| | 2 months | 99.98 | 99.97 | 0 | 0.01 | Unchanged | Unchanged |
| 40 °C/75% RH | 1 week | 99.97 | 99.97 | 0.01 | 0.01 | Unchanged | Unchanged |
| | 2 weeks | 99.98 | 99.99 | 0 | -0.01 | Unchanged | Unchanged |
| | 1 month | 99.98 | 99.98 | 0 | 0 | Unchanged | Unchanged |
| | 2 months | 99.97 | 99.96 | 0.01 | 0.02 | Unchanged | Unchanged |
| 80 °C | 1 d | 99.98 | 99.97 | 0 | 0.01 | Unchanged | Unchanged |
| Lighting | 10 d | 99.98 | 99.98 | 0 | 0 | Unchanged | Unchanged |
| Lighting control | | 99.98 | 99.98 | 0 | 0 | Unchanged | Unchanged |

As can be seen from Table 1, both the crystal forms A and B show almost no degradation under the test conditions. As can be seen from the overlay of XRPD forms in FIGS. 15 and 16, the crystal forms A and B have not changed under the test conditions. The above results indicate that the crystal forms A and B have good stability, are not prone to crystal transformation, and can be used as active pharmaceutical ingredients to produce formulation products.

Example 6: Evaluation of Stability of Crystal Forms A and B in Aqueous Medium

2 mL of 5 mg/mL suspension of crystal form A (prepared according to the preparation method 1 of Example 1) and 2 mL of 5 mg/mL suspension of crystal form B (prepared according to the method of Example 2) were prepared respectively: adding the samples of crystal forms A and B to FaSSIF, FeSSIF, SGF, water, and buffers of pH 1.2 (potassium chloride), pH 3.0 (potassium biphthalate), pH 4.5 (sodium acetate trihydrate), pH 6.8 (potassium dihydrogen phosphate), and pH 7.4 (potassium dihydrogen phosphate), respectively, stirring in a water bath at 37 °C, and after 24 h, carrying out pH detection for filtrates and XRPD testing for solids. The test results are shown in Table 2 and FIGS. 17 and 18.

Table 2: Results of Stability of Crystal Forms A and B in Aqueous Medium at 37 °C

| Medium | Time | XRPD | | Medium | Time | XRPD | |
|---|---|---|---|---|---|---|---|
| | | Crystal form A | Crystal form B | | | Crystal form A | Crystal form B |
| pH 1.2 | 24 h | Crystal form B | Unchanged | Water | 24 h | Crystal form B | Unchanged |
| pH 3.0 | 24 h | Crystal form B | Unchanged | FaSSIF | 24 h | Unchanged | Unchanged |
| pH 4.5 | 24 h | Crystal form B | Unchanged | FeSSIF | 24 h | Unchanged | Unchanged |
| pH 6.8 | 24 h | Crystal form B | Unchanged | FeSSIF | 24 h | Unchanged | Unchanged |
| pH 7.4 | 24 h | Crystal form B | Unchanged | / | / | / | / |

As can be seen from Table 2 and FIGS. 17 and 18, the crystal form A is transformed to the crystal form B after 24 hours in buffers of different pHs and water, but without crystal form transformation in simulated biological media. The crystal form B shows no change in all media.

Example **7:** Impacts of Different Crystal Forms on Preparation of Solid Dispersion

Under the same hot-melt extrusion conditions, the contents of related substance D1 in the solid dispersions prepared from the crystal forms A (prepared according to the preparation method 1 of Example 1) and B (prepared according to the preparation method of Example 2) were investigated, respectively. The structure of the related substance D1 is represented by formula (II).

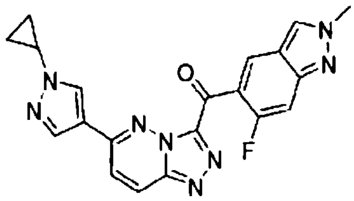

(II)

Preparation of solid dispersion by hot-melt extrusion: the active pharmaceutical ingredient of crystal form A (prepared according to the preparation method 1 of Example 1) or B (prepared according to the method of Example 2) and Povidone K30 were weighed respectively and then evenly mixed at a ratio of 1:3; after the temperature of each temperature zone for hot-melt extraction reached 130 °C, 150 °C, 180 °C, 195 °C, 200 °C, 200 °C, 200 °C, and 200 °C and balanced for 15 min, the mixture was added to the hopper of an extruder; and the resulting solids were collected and left to cool at room temperature, and then tested for the content of the related substance D1, respectively. The results are shown in Table 3.

Table 3: Results of Prepared Samples of Crystal Forms A and B

| Test item | Active pharmaceutical ingredient | |
|---|---|---|
| | Crystal form A | Crystal form B |
| Content of related substance D1 in starting material | Not detected | Not detected |
| Content of active pharmaceutical ingredient in sample | 101.6% | 101.8% |
| Content of related substance D1 in sample | 0.08% | 0.05% |

Not detected: the detected content of related substance D1 was < 0.0045%.

As can be seen from Table 3, for the solid dispersions prepared from the crystal forms A and B with the hot-melt extrusion process respectively, no significant difference is observed in the content of active pharmaceutical ingredients in the samples obtained, but different crystal forms show different impurities in the prepared samples. The content of related substance D1 in the sample prepared using the crystal form B is lower than that in the sample prepared using the crystal form A. Therefore, the crystal form B is superior to the crystal form A in sample preparation.

Example **8:** Impacts of Different Crystal Forms on Sample Preparation Process

The active pharmaceutical ingredient of crystal form A (prepared according to the preparation method 1 of Example 1) or B (prepared according to the method of Example 2) and Povidone K30 were weighed respectively and then evenly mixed at a ratio of 1:3; after the temperature of each temperature zone for hot-melt extraction reached 130 °C, 150 °C, 180 °C, 195 °C, 200 °C, 200 °C, 200 °C, and 200 °C and balanced for 15 min, the mixture was added to the hopper of an extruder; and the resulting solids were collected and left at room temperature. The entire process was observed. The results are shown in Table 4.

Table 4: Sample Preparation Processes of Different Crystal Forms

| Observation item | Active pharmaceutical ingredient | |
|---|---|---|
| | Crystal form A | Crystal form B |
| Premix fluidity | Poor | Good |
| Feeding state | Lumpy | Homogeneous powder |
| Feeding speed | Slow | Fast |
| Availability for continuous sample preparation | Unavailable for continuous sample preparation, with bridging occurring at the feed port, in which case a stainless steel spoon was required to assist in feeding and clogging occurred if feeding continued. | Available for continuous sample preparation without clogging, allowing for long-term continuous hot-melt extrusion. |
| Sample acceptability | Unacceptable | Acceptable |

As can be seen from Table 4, during the sample preparation of the crystal form A, agglomeration occurs during feeding, and even clogging occurs in case of low feeding speed, leading to unacceptable final samples. In the case of the crystal form B, the premix is dispersed during feeding, allowing for high feeding speed, continuous sample preparation without clogging, and long-term continuous hot-melt extrusion, leading to acceptable prepared samples.

Example **9:** Hygroscopic Weight Gain Test of Crystal Form B

2.6 g of the crystal form B (prepared according to the method of Example 2) was weighed, placed in an open weighing bottle, and evenly spread (at the thickness of ≤ 3 mm), and the above process was repeated to prepare a total of 6 samples. On Days 0, 20 and 25, the samples were set out respectively, with the temperature and humidity conditions controlled to 25 ± 2 °C/75 ± 5% RH and 25 ± 2 °C/90 ± 5% RH. All samples were sampled and weighed on Day 30 to calculate the hygroscopic weight gain (%). The results are shown in Table 5.

$$\text{Weight gain (\%)} = (m_3 - m_2)/(m_2 - m_1)*100\%$$

Table 5: Hygroscopic weight gain results of crystal form B under high humidity conditions

| Conditions | 25 + 2°C/90 + 5% RH | | | 25 ± 2°C/75 + 5% RH | | |
|---|---|---|---|---|---|---|
| | 5 days | 10 days | 30 days | 5 days | 10 days | 30 days |
| Weighing bottle ($m_1$, g) | 20.94383 | 21.60791 | 21.84344 | 21.37522 | 21.12806 | 20.82797 |
| Initial weight ($m_2$, g) (sample + weighing bottle) | 23.54082 | 24.21364 | 24.45472 | 23.98139 | 23.74307 | 23.47962 |
| Weight after standing ($m_3$, g) (sample + weighing bottle) | 23.57464 | 24.24852 | 24.49111 | 24.00855 | 23.77380 | 23.51043 |
| Hygroscopic weight gain (%) | 1% | 1% | 1% | 1% | 1% | 1% |

As can be seen from Table 5, when held under high humidity conditions for 5 days, 10 days and 30 days respectively, the crystal form B shows the hygroscopic weight gain of approximately 1%, which is less than 2%, indicating that the crystal form B has slight hygroscopicity.

In summary, the free base crystal forms A and B of the present application have good stability and slight hygroscopicity, which is conducive to storage. The obtained crystal forms have uniform particle size, which is conducive to mixing with excipients. In addition, the melting points of the crystal forms A and B are approximately 222 °C and 203 °C, respectively, and the melting point of the crystal form B is lower than that of the crystal form A. Compared with the free base crystal form A, the free base crystal form B of the present application is better when used to prepare samples through the hot-melt extrusion process, and the obtained solid dispersion has a relatively low impurity content of related substance, which is more conducive to the quality of pharmaceuticals. Moreover, the crystal form B is granular, which is more conducive to uniform mixing with excipients, resulting in reduced friction during mixing and higher smoothness during preparation of the solid dispersion. Therefore, the crystal form B has a significant advantage over the crystal form A.

## Claims

1. A crystal form B of 6-(1-cyclopropyl-1-hydro-pyrazol-4-yl)-3-(difluoro(6-fluoro-2-methyl-(dihydro-indazol-5-yl) methyl)-[1,2,4]triazolo[4,3-b]pyridazine represented by formula (I), wherein with Cu-Kα radiation, the crystal form B has characteristic peaks at 2θ values of 8.38°, 15.99°, 18.73°, 24.86°, 28.96°, and 29.83° in a X-ray powder diffraction pattern expressed in 2θ angles, with a 2θ error range of ± 0.2°,

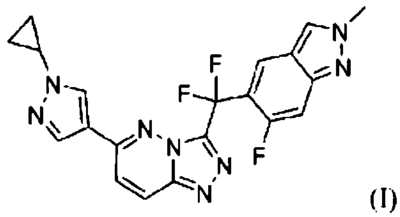

(I)

2. The crystal form B according to claim 1, wherein the X-ray powder diffraction pattern of the crystal form B shows characteristic peaks at 2θ values of 8.38°, 15.57°, 15.99°, 18.73°, 24.86°, 25.30°, 28.96°, and 29.83°, with a 2θ error range of ± 0.2°; and
preferably, the X-ray powder diffraction pattern of the crystal form B is shown in FIG. 5.

3. The crystal form B according to claim 1 or 2, wherein a thermogravimetric analysis (TGA) thermogram of the crystal form B shows a weight loss of up to 0.50% from room temperature to 200 °C;

   preferably, a TGA thermogram of the crystal form B is shown in FIG. 6;
   preferably, a differential scanning calorimetry (DSC) thermogram of the crystal form B shows an endothermic peak value at 203.00 °C; and
   preferably, a DSC thermogram of the crystal form B is shown in FIG. 7.

4. A preparation method for the crystal form B according to any one of claims 1 to 3, comprising:

   adding the compound of formula (I) to a mixture of a normal solvent B1 and an anti-solvent B2, heating until dissolution, filtering and cooling, cultivating crystals, adding the anti-solvent B2, and cooling to 0 °C to obtain the crystal form B, wherein
   preferably, the normal solvent B1 is acetone,
   preferably, the anti-solvent B2 is water,
   preferably, a heating temperature is 0 °C to 60 °C, preferably 60 °C,
   preferably, before cultivating the crystals, a cooling temperature is 40 °C to 0 °C, preferably 30 °C, and
   preferably, in the mixture of the normal solvent B1 and the anti-solvent B2, a volume ratio of the normal solvent B1 to the anti-solvent B2 is 4/1 to 1/1, preferably 3/1; or
   adding the compound of formula (I) to a mixture of a normal solvent B1 and an anti-solvent B2, heating until dissolution, filtering and cooling to obtain the crystal form B, wherein
   preferably, the normal solvent B1 is acetone,
   preferably, the anti-solvent B2 is water,
   preferably, a heating temperature is 0 °C to 60 °C, preferably 60 °C,

preferably, a cooling temperature is 0 °C to 30 °C, preferably 0 °C to 10 °C, and
preferably, in the mixture of the normal solvent B1 and the anti-solvent B2, a volume ratio of the normal solvent B1 to the anti-solvent B2 is 3/1 to 4/1, preferably 4/1.

**5.** A crystal form A of 6-(1-cyclopropyl-1-hydro-pyrazol-4-yl)-3-(difluoro(6-fluoro-2-methyl-(dihydro-indazol-5-yl) methyl)[1,2,4]triazolo[4,3-b]pyridazine represented by formula (I), wherein with Cu-Kα radiation, the crystal form A has characteristic peaks at 2θ values of 7.47°, 10.30°, 12.47°, 14.18°, 17.19°, 24.25°, and 25.43° in a X-ray powder diffraction pattern expressed in 2θ angles, with a 2θ error range of ± 0.2°,

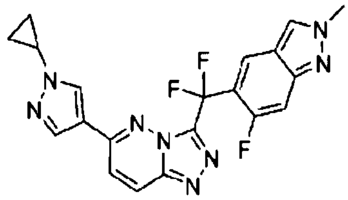

(I).

**6.** The crystal form A according to claim 5, wherein the X-ray powder diffraction pattern of the crystal form A shows characteristic peaks at 2θ values of 7.47°, 10.30°, 12.47°, 14.18°, 17.19°, 17.54°, 17.85°, 18.15°, 20.39°, 24.25°, 25.43°, 25.97° and 26.43°, with a 2θ error range of ± 0.2°; and
preferably, the X-ray powder diffraction pattern of the crystal form A is shown in FIG. 1.

**7.** The crystal form A according to claim 5 or 6, wherein a thermogravimetric analysis (TGA) curve of the crystal form A shows no weight loss from room temperature to a melting point;

preferably, a TGA thermogram of the crystal form A is shown in FIG. 2;
preferably, a differential scanning calorimetry (DSC) curve of the crystal form A shows an endothermic peak value at 222.27 °C; and
preferably, a DSC thermogram of the crystal form A is shown in FIG. 3.

**8.** A preparation method for the crystal form A according to any one of claims 5 to 7, comprising:

dissolving the compound of formula (I) in a normal solvent A1 at 0 °C to 70 °C, filtering, adding the resulting clear filtrate dropwise to an anti-solvent A2 to obtain the crystal form A, wherein
preferably, the normal solvent A1 is acetic acid,
preferably, the anti-solvent A2 is ethanol,
preferably, a volume ratio of the normal solvent A1 to the anti-solvent A2 is 1/6 to 1/1, more preferably 1/4 or 1/3, and
preferably, a temperature at which the compound of formula (I) is dissolved in the normal solvent A1 is 20 °C to 40 °C, more preferably 35 °C, to enable clear dissolution of the compound of formula (I) in the normal solvent A1; or
dissolving the compound of formula (I) in a normal solvent A1, filtering, and adding an anti-solvent A2 dropwise to the resulting clear filtrate to obtain the crystal form A; and
preferably, the normal solvent A1 is acetic acid,
preferably, the anti-solvent A2 is ethanol,
preferably, a volume ratio of the normal solvent A1 to the anti-solvent A2 is 1/6 to 1/1, more preferably 1/4 or 1/3, and
preferably, a temperature at which the compound of formula (I) is dissolved in the normal solvent A1 is 20 °C to 50 °C, more preferably 35 °C; or
dissolving the compound of formula (I) in a certain volume of solvent or mixed solvent, heating until complete dissolution, and filtering and slow cooling to obtain the crystal form A, and
preferably, a warming temperature is 20 °C to 70 °C, more preferably 50 °C,
preferably, the solvent is acetonitrile, methanol or acetic acid,
preferably, the mixed solvent is a mixture of acetic acid and ethanol, and
preferably, a volume ratio of the mixed solvent is 1/6 to 1/1, more preferably 1/4 or 1/3.

**9.** A pharmaceutical preparation or a pharmaceutical composition, comprising the crystal form B according to any one of claims 1 to 3 or the crystal form A according to any one of claims 5 to 7, and a pharmaceutically acceptable carrier and/or vehicle; and

preferably, the pharmaceutical preparation or the pharmaceutical composition is a tablet, capsule, pill, granule, powder, suppository, injection, solution, suspension, ointment, patch, lotion, drop, liniment, or spray.

**10.** Use of the crystal form B according to any one of claims 1 to 3, or the crystal form A according to any one of claims 5 to 7, or the pharmaceutical preparation or the pharmaceutical composition according to claim 9 in preparation of a medicament for treating c-Met abnormality-mediated diseases, wherein

preferably, the c-Met abnormality-mediated diseases are tumor diseases; and
preferably, the tumor diseases comprise: head and neck cancer, nasopharyngeal cancer, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin lymphoma, stomach cancer, lung cancer, liver cancer, gastrointestinal stromal tumor, thyroid cancer, squamous cell carcinoma, cholangiocarcinoma, endometrial cancer, multiple myeloma or mesothelioma, atherosclerosis, or pulmonary fibrosis.

**11.** A method for treating c-Met abnormality-mediated diseases, comprising administering the crystal form B according to any one of claims 1 to 3, or the crystal form A according to any one of claims 5 to 7, or the pharmaceutical preparation or the pharmaceutical composition according to claim 9 to a subject in need thereof, wherein

preferably, the c-Met abnormality-mediated diseases are tumor diseases; and
preferably, the tumor diseases comprise: head and neck cancer, nasopharyngeal cancer, melanoma, bladder cancer, esophageal cancer, anaplastic large cell lymphoma, renal cancer, breast cancer, colorectal cancer, ovarian cancer, cervical cancer, pancreatic cancer, glioma, glioblastoma, prostate cancer, leukemia, lymphoma, non-Hodgkin lymphoma, stomach cancer, lung cancer, liver cancer, gastrointestinal stromal tumor, thyroid cancer, squamous cell carcinoma, cholangiocarcinoma, endometrial cancer, multiple myeloma or mesothelioma, atherosclerosis, or pulmonary fibrosis.

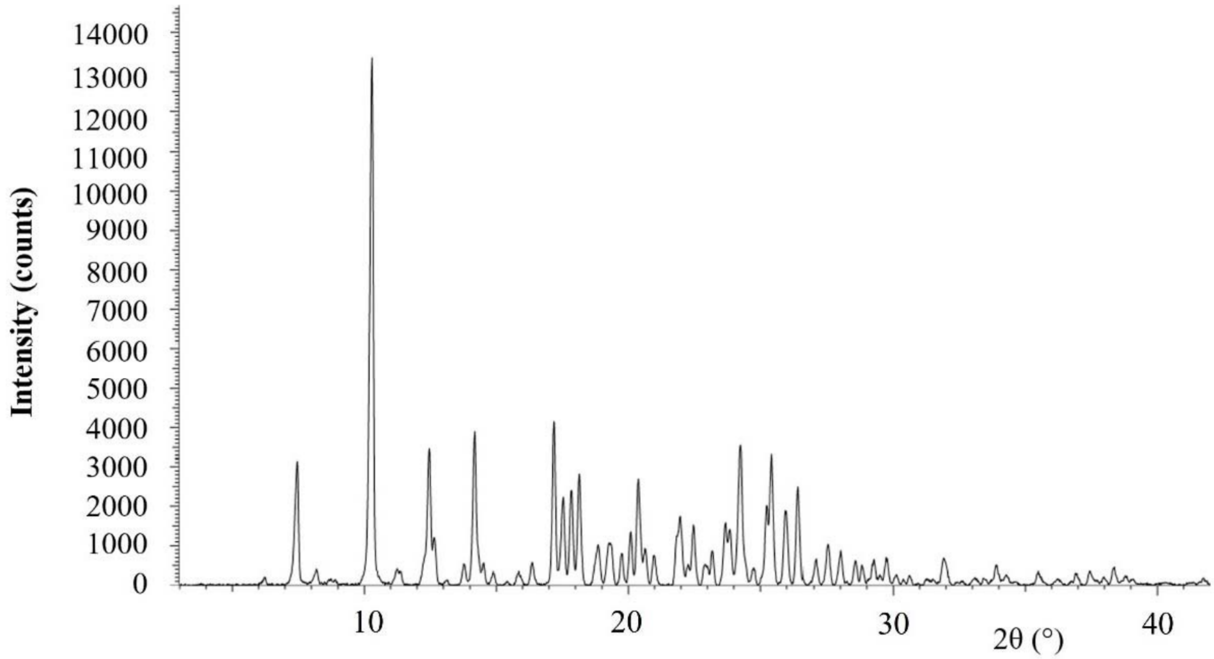
14000
13000
12000
11000
10000
9000
8000
7000
6000
5000
4000
3000
2000
1000
0
Intensity (counts)
10
20
30
40
2θ (°)

FIG. 1

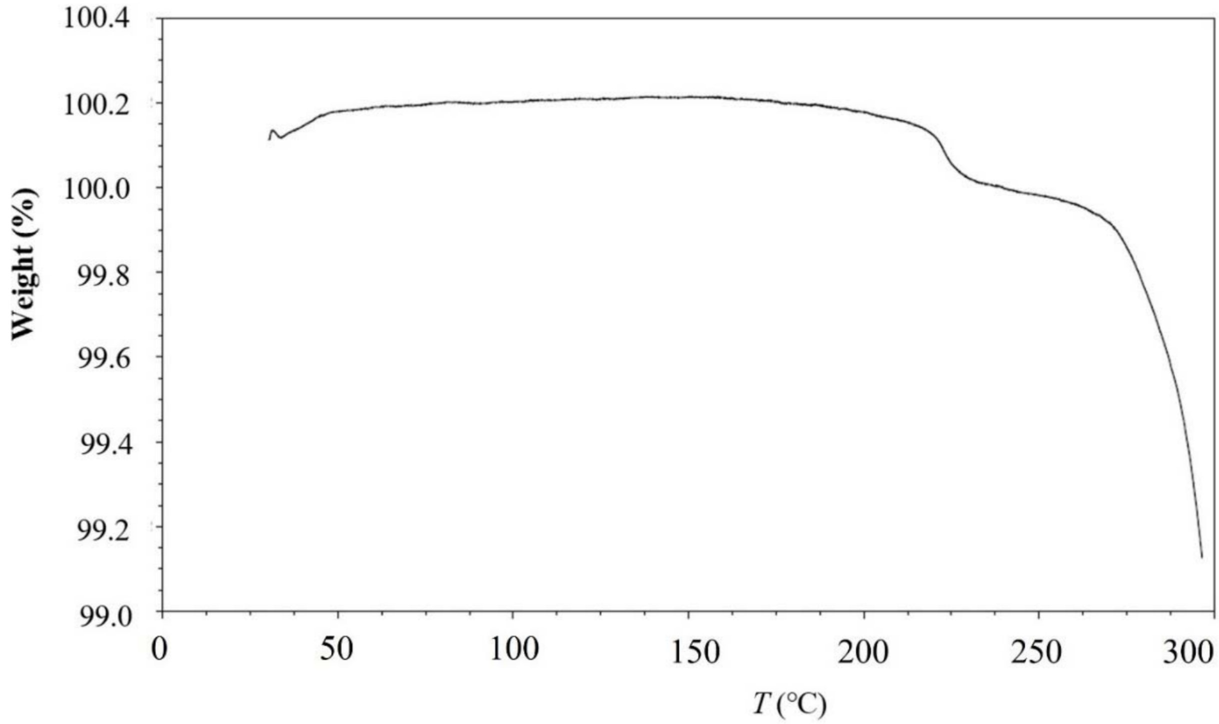
100.4
100.2
100.0
99.8
99.6
99.4
99.2
99.0
Weight (%)
0
50
100
150
200
250
300
T (°C)

FIG. 2

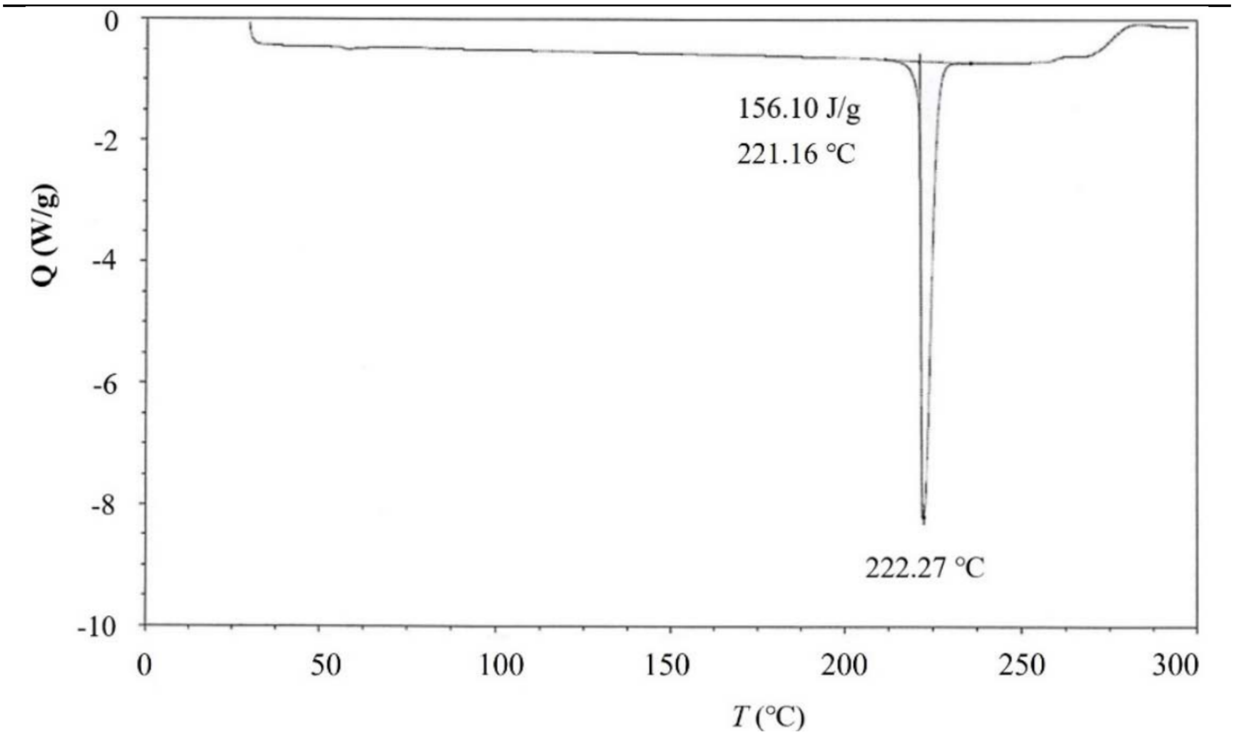
0
-2
-4
-6
-8
-10
Q (W/g)
156.10 J/g
221.16 °C
222.27 °C
0
50
100
150
200
250
300
T (°C)

FIG. 3

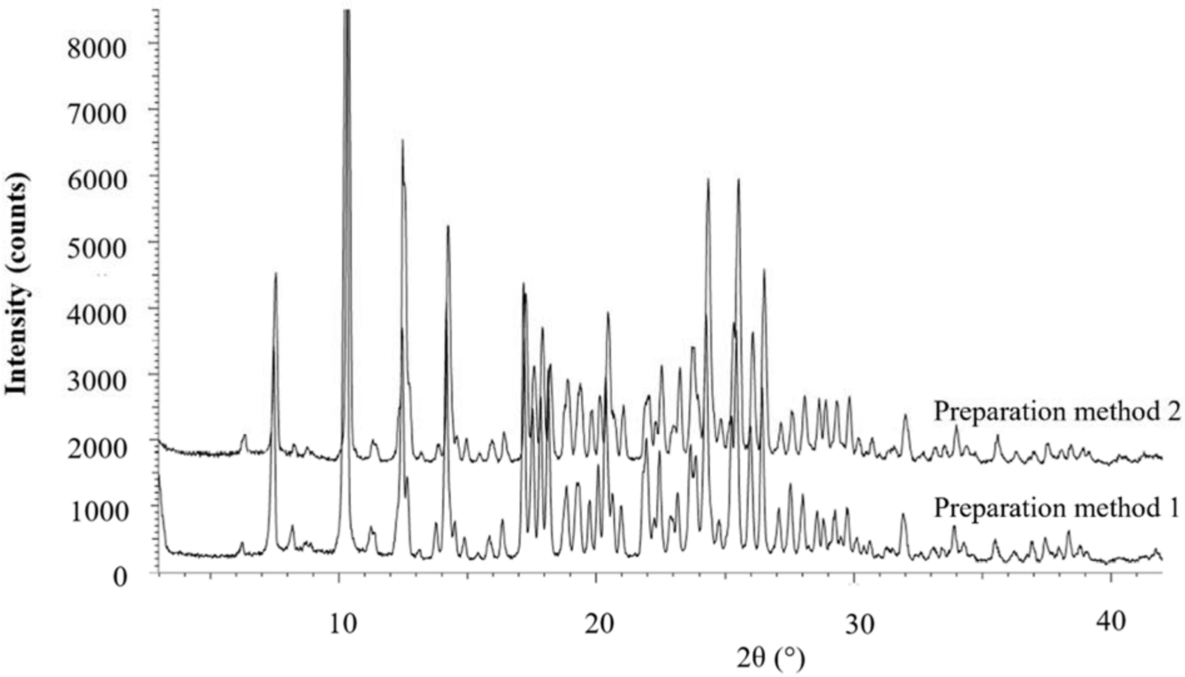
8000
7000
6000
5000
4000
3000
2000
1000
0
Intensity (counts)
Preparation method 2
Preparation method 1
10
20
30
40
2θ (°)

FIG. 4

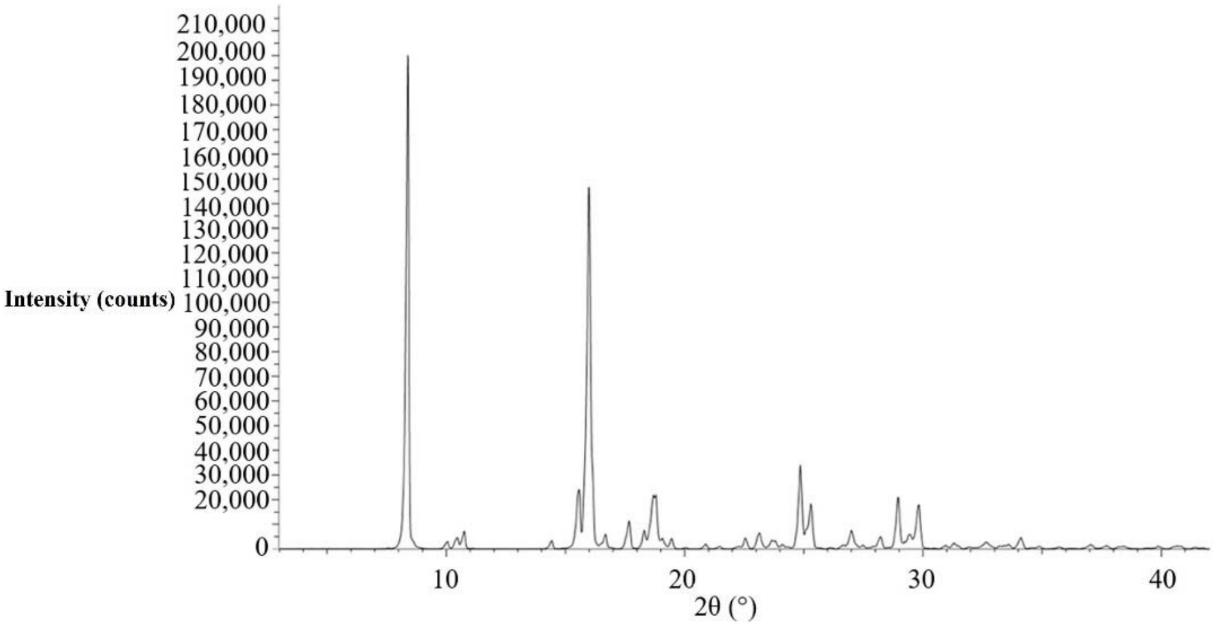
Intensity (counts)
210,000
200,000
190,000
180,000
170,000
160,000
150,000
140,000
130,000
120,000
110,000
100,000
90,000
80,000
70,000
60,000
50,000
40,000
30,000
20,000
0
10
20
30
40
2θ (°)

FIG. 5

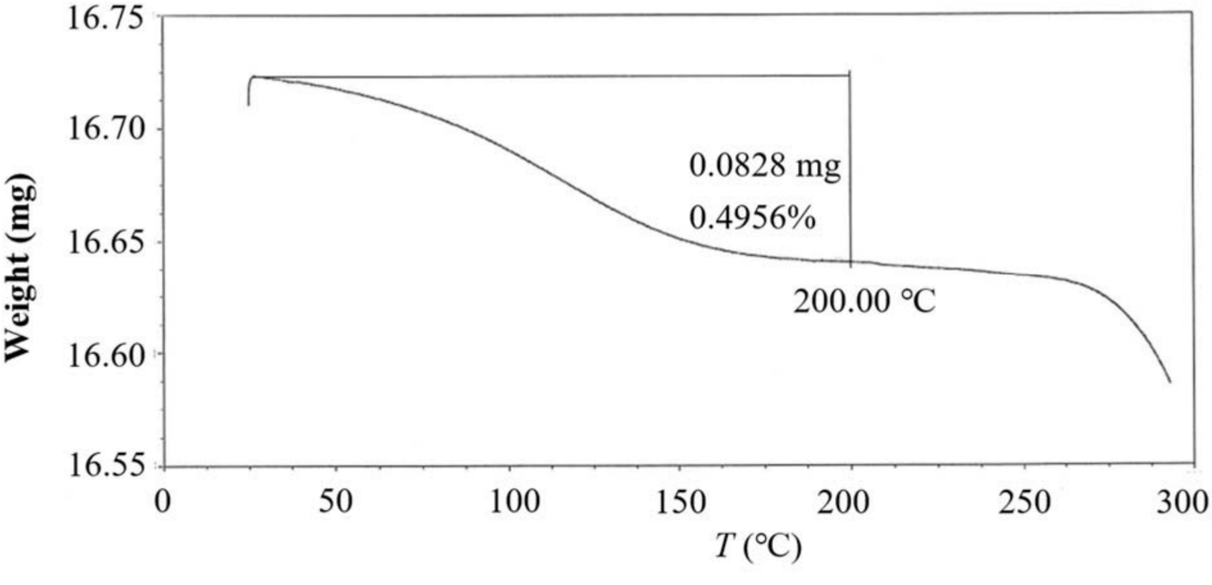
Weight (mg)
16.75
16.70
16.65
16.60
16.55
0.0828 mg
0.4956%
200.00 °C
0
50
100
150
200
250
300
T (°C)

FIG. 6

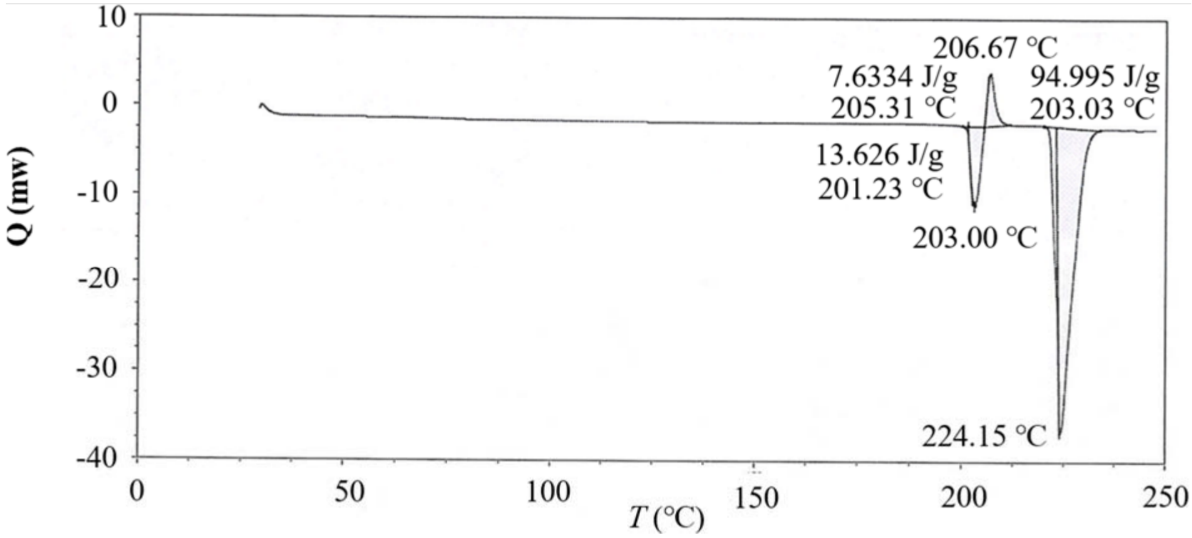


FIG. 7

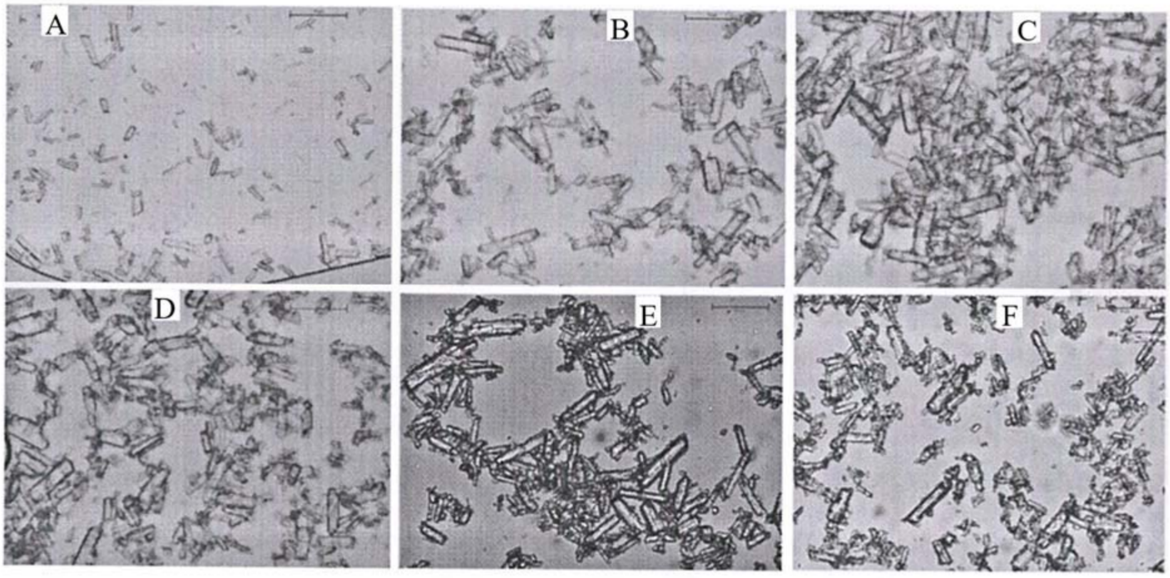


FIG. 8

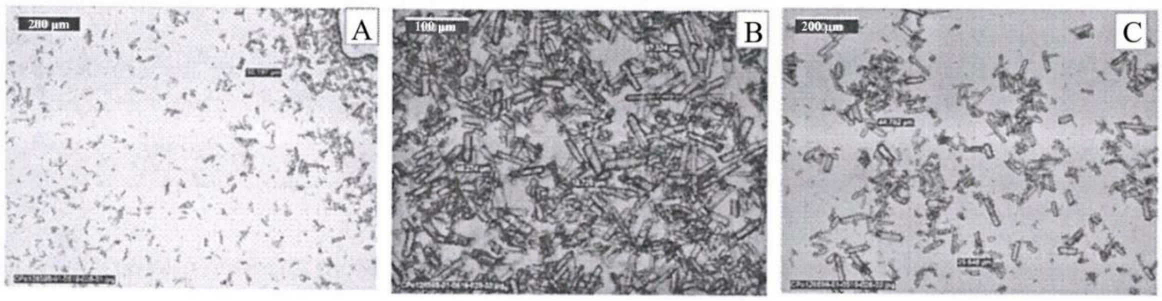


FIG. 9

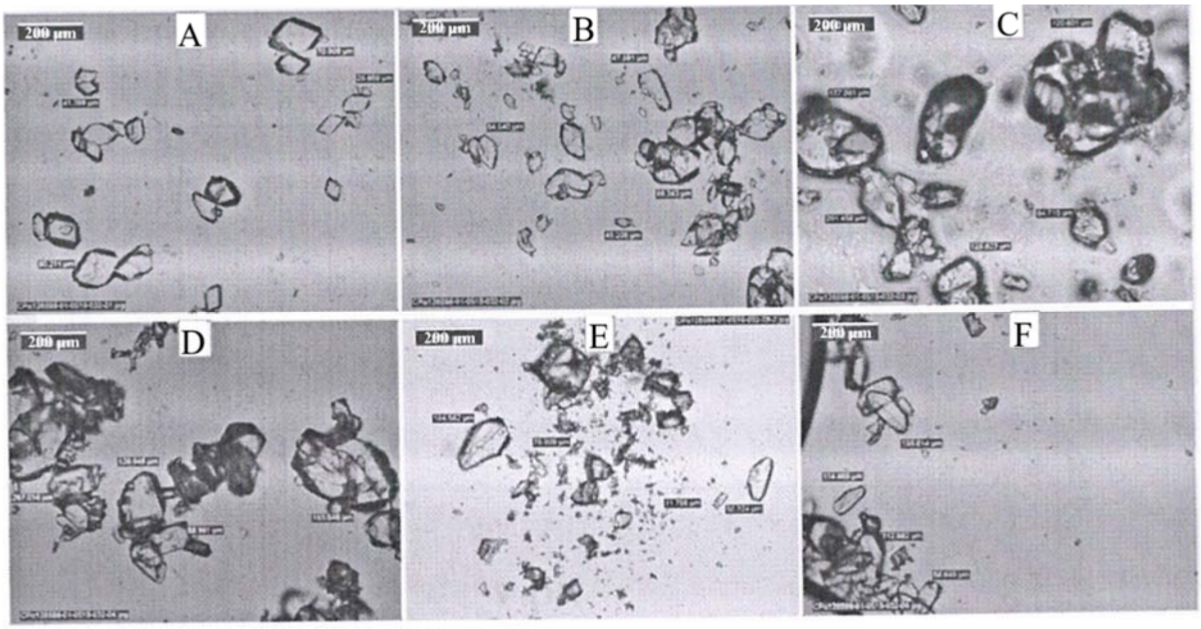
A
B
C
D
E
F
200 μm

FIG. 10

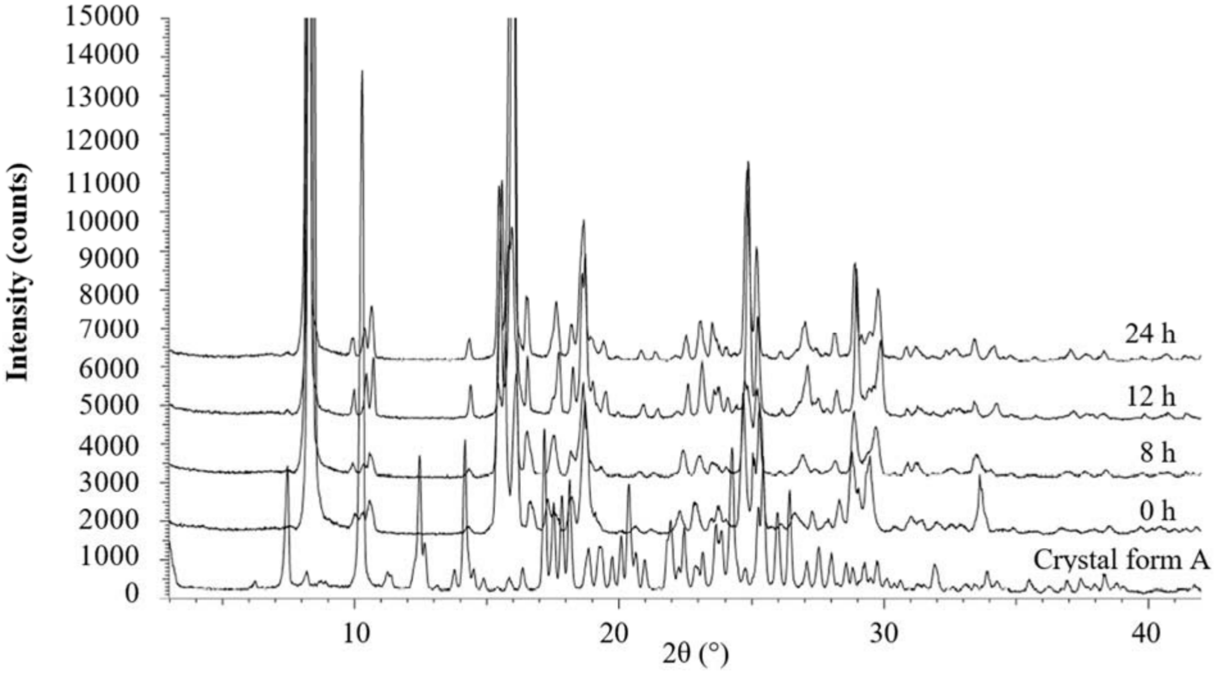
Intensity (counts)
2θ (°)
24 h
12 h
8 h
0 h
Crystal form A
0
1000
2000
3000
4000
5000
6000
7000
8000
9000
10000
11000
12000
13000
14000
15000
10
20
30
40

FIG. 11

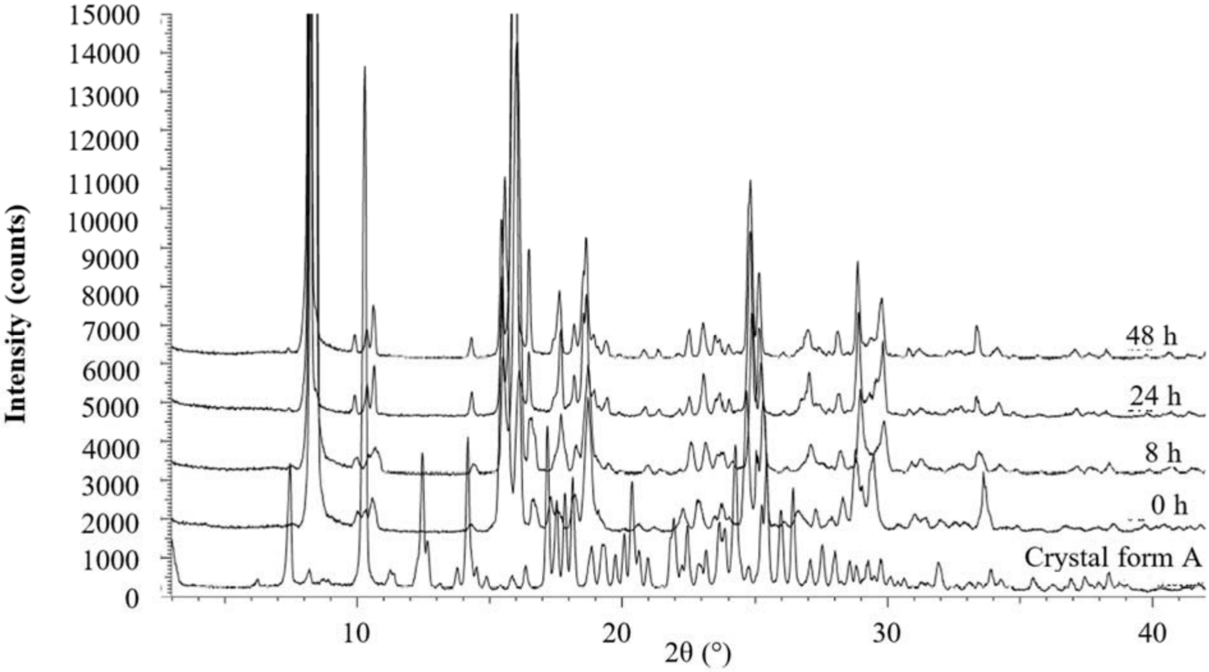
15000
14000
13000
12000
11000
10000
9000
8000
7000
6000
5000
4000
3000
2000
1000
0
Intensity (counts)
48 h
24 h
8 h
0 h
Crystal form A
10
20
30
40
2θ (°)

FIG. 12

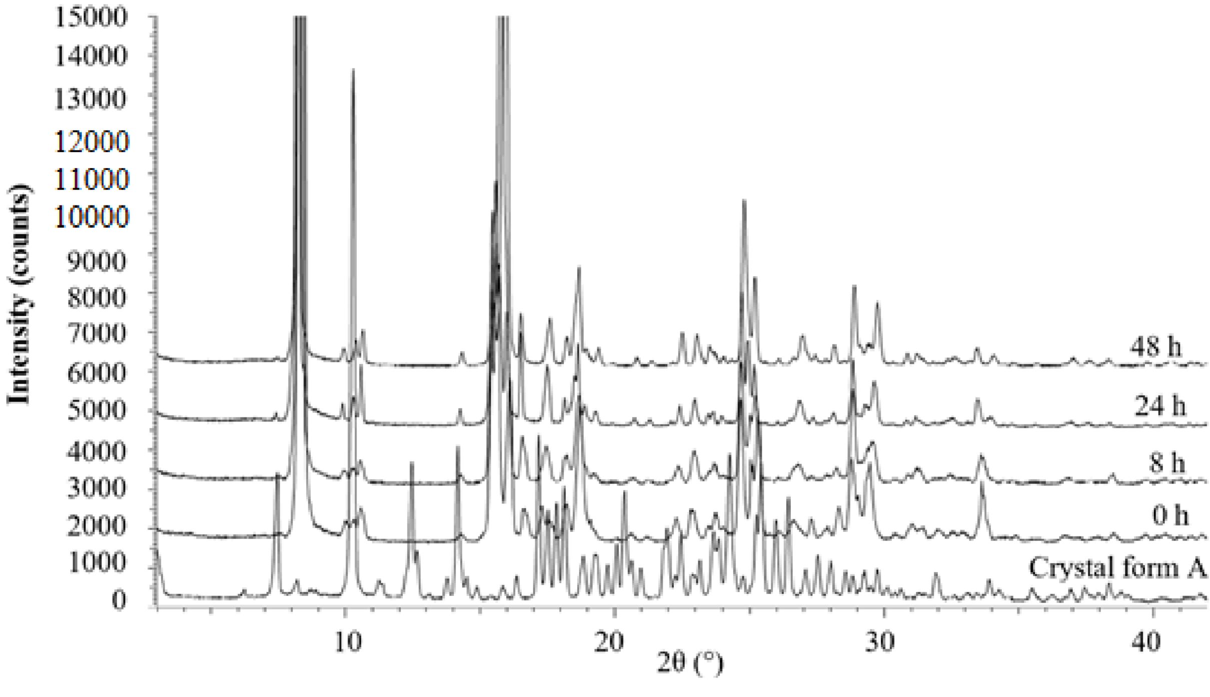
15000
14000
13000
12000
11000
10000
9000
8000
7000
6000
5000
4000
3000
2000
1000
0
Intensity (counts)
48 h
24 h
8 h
0 h
Crystal form A
10
20
30
40
2θ (°)

FIG. 13

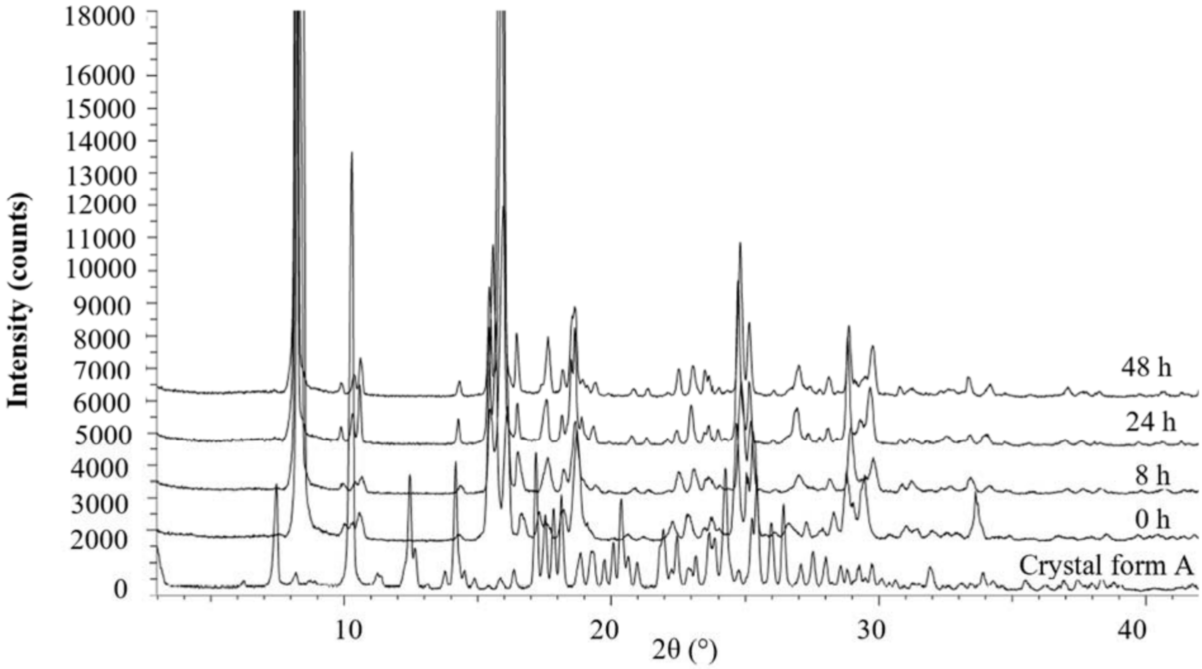
Intensity (counts)
18000
16000
15000
14000
13000
12000
11000
10000
9000
8000
7000
6000
5000
4000
3000
2000
0
10
20
30
40
2θ (°)
48 h
24 h
8 h
0 h
Crystal form A

FIG. 14

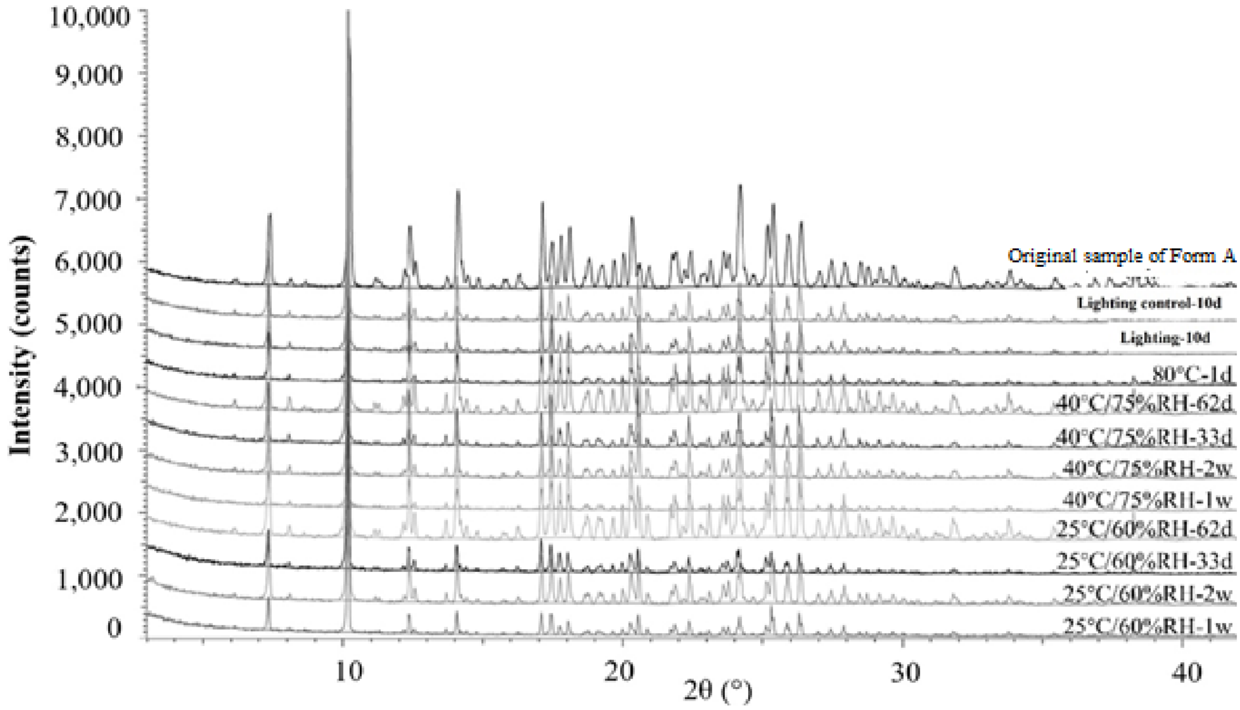
Intensity (counts)
10,000
9,000
8,000
7,000
6,000
5,000
4,000
3,000
2,000
1,000
0
10
20
30
40
2θ (°)
Original sample of Form A
Lighting control-10d
Lighting-10d
80°C-1d
40°C/75%RH-62d
40°C/75%RH-33d
40°C/75%RH-2w
40°C/75%RH-1w
25°C/60%RH-62d
25°C/60%RH-33d
25°C/60%RH-2w
25°C/60%RH-1w

FIG. 15

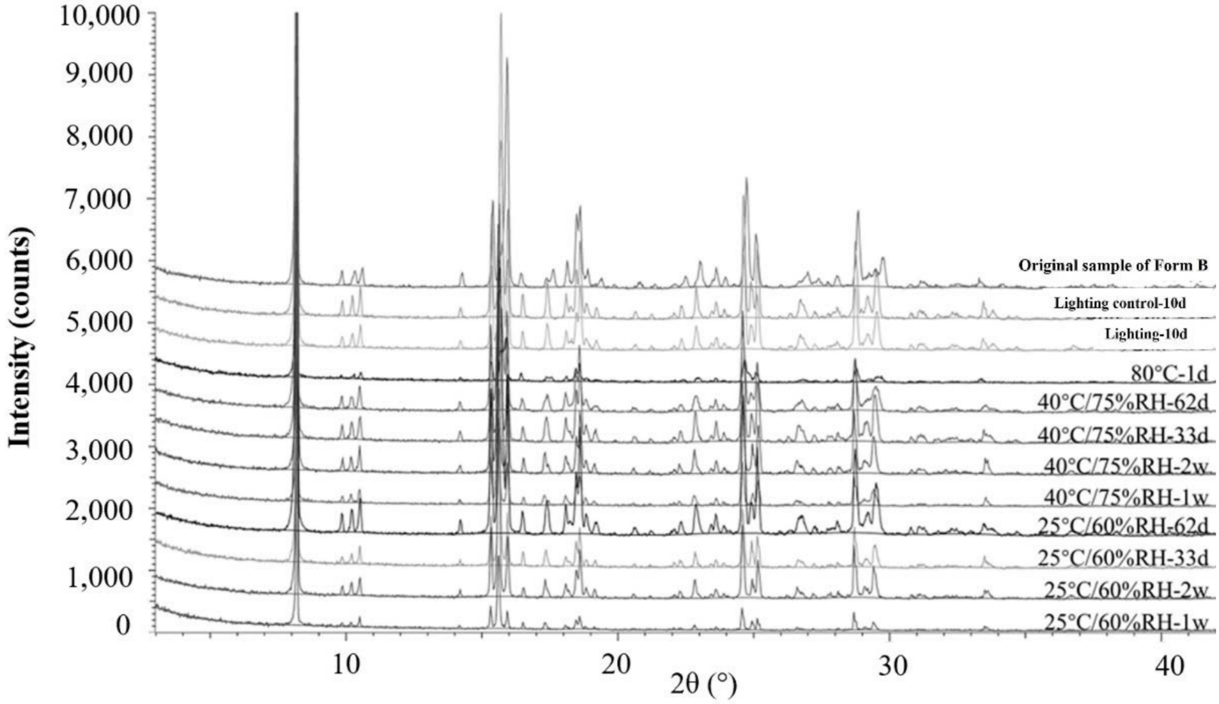
Intensity (counts)
10,000
9,000
8,000
7,000
6,000
5,000
4,000
3,000
2,000
1,000
0
10
20
30
40
2θ (°)
Original sample of Form B
Lighting control-10d
Lighting-10d
80°C-1d
40°C/75%RH-62d
40°C/75%RH-33d
40°C/75%RH-2w
40°C/75%RH-1w
25°C/60%RH-62d
25°C/60%RH-33d
25°C/60%RH-2w
25°C/60%RH-1w

FIG. 16

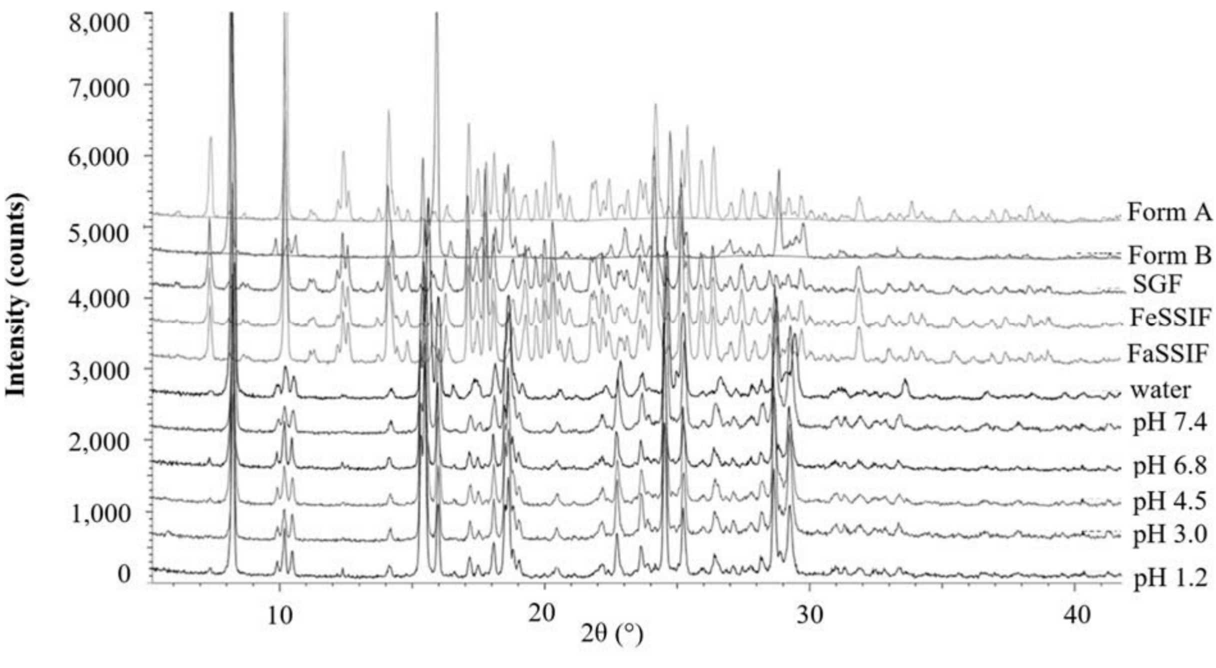
Intensity (counts)
8,000
7,000
6,000
5,000
4,000
3,000
2,000
1,000
0
10
20
30
40
2θ (°)
Form A
Form B
SGF
FeSSIF
FaSSIF
water
pH 7.4
pH 6.8
pH 4.5
pH 3.0
pH 1.2

FIG. 17

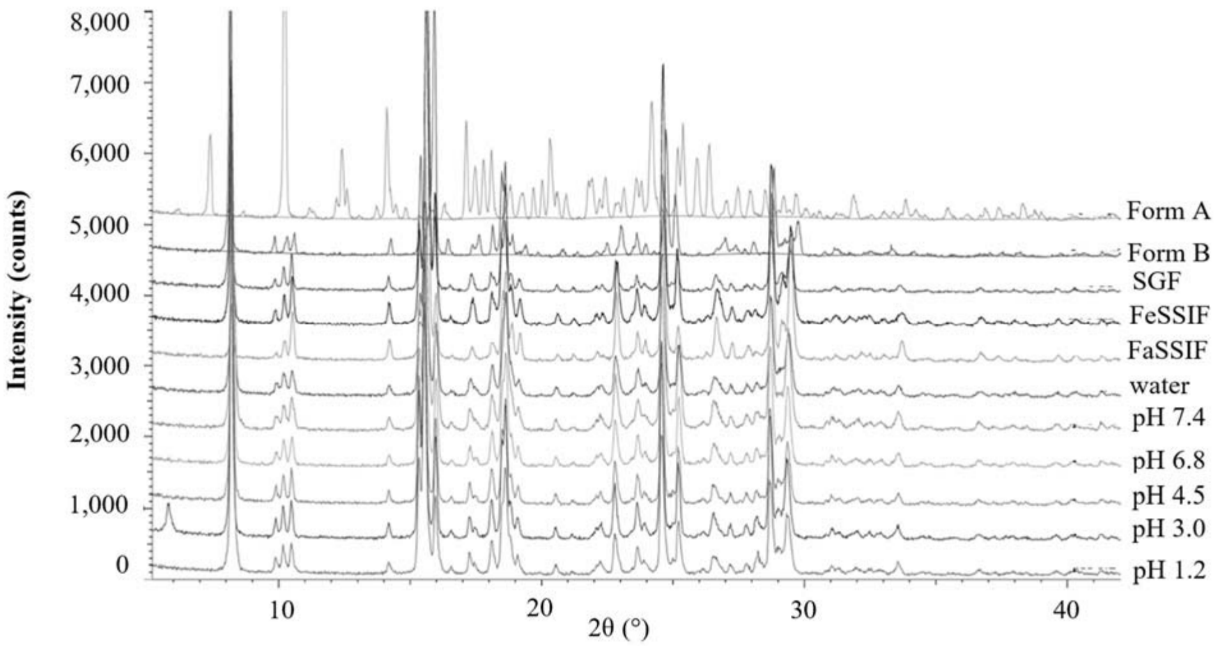

8,000
7,000
6,000
5,000
4,000
3,000
2,000
1,000
0
Intensity (counts)
10
20
30
40
2θ (°)
Form A
Form B
SGF
FeSSIF
FaSSIF
water
pH 7.4
pH 6.8
pH 4.5
pH 3.0
pH 1.2


FIG. 18

## INTERNATIONAL SEARCH REPORT

International application No.
**PCT/CN2023/105714**

**A. CLASSIFICATION OF SUBJECT MATTER**

C07D487/04(2006.01)i; C07D471/04(2006.01)i; A61K31/5025(2006.01)i; A61K31/437(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D; A61K; A61P

Documentation searched other than minimum documentation to the extent that such documents are included in the fields searched

Electronic data base consulted during the international search (name of data base and, where practicable, search terms used)

DWPI, CNTXT, STNext, CNKI, EPTXT, USTXT, 万方, WANFANG, 百度学术, Baidu Scholar, 读秀, DUXIU, 北京浦润奥生物, 中美冠科生物, 宋伟周, 李刚, 张培龙, 石和鹏, 仲伯禹, 晶型, 结晶, 伯瑞替尼, 哒嗪, 三唑, 吲唑, 肿瘤, 癌症, 非小细胞肺癌, MET, bozitinib, PLB, 1001, crystal, pyridazine, triazole, indazole, tumor, cancer, NSCLC, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 103122000 A (CROWN BIOSCIENCE (TAICANG) INC.) 29 May 2013 (2013-05-29) description, paragraphs [0010]-[0020] and [0206]-[0208] | 1-11 |
| A | CN 116283997 A (YAOKANG ZHONGTUO (JIANGSU) PHARMACEUTICAL TECHNOLOGY CO., LTD.) 23 June 2023 (2023-06-23) description, paragraphs [0006]-[0023] | 1-11 |
| A | CN 105326793 A (CROWN BIOSCIENCE (TAICANG) INC. et al.) 17 February 2016 (2016-02-17) description, paragraphs [0011]-[0033] | 1-11 |
| A | WO 2021222045 A1 (APOLLOMICS INC.) 04 November 2021 (2021-11-04) description, paragraphs [0041]-[0043] | 1-11 |
| A | CN 101374843 A (JANSSEN PHARMACEUTICA NV) 25 February 2009 (2009-02-25) claims 1-48 | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 August 2023** | **17 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/105714**

**C. DOCUMENTS CONSIDERED TO BE RELEVANT**

| Category* | Citation of document, with indication, where appropriate, of the relevant passages | Relevant to claim No. |
|---|---|---|
| A | WANG, Chaofan et al. "Targeting MET: Discovery of Small Molecule Inhibitors as Non-Small Cell Lung Cancer Therapy" *Journal of Medicinal Chemistry,* Vol. vol. 66, 01 June 2023 (2023-06-01), pp. 7670-7697 page 7679, figure 12 | 1-11 |

Form PCT/ISA/210 (second sheet) (July 2022)

**INTERNATIONAL SEARCH REPORT**

International application No.
**PCT/CN2023/105714**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:

The subject matter of claim 11 is "a method for treating c-Met abnormal mediated diseases", which relates to a living human or animal body, and falls within disease treatment methods. The present report is provided on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/105714**

| Patent document cited in search report | Publication date (day/month/year) | Patent family member(s) | Publication date (day/month/year) |
|---|---|---|---|
| CN 103122000 A | 29 May 2013 | HK 1210154 A1 | 15 April 2016 |
| | | US 2015218171 A1 | 06 August 2015 |
| | | US 9695175 B2 | 04 July 2017 |
| | | WO 2014032498 A1 | 06 March 2014 |
| | | JP 2015526486 A | 10 September 2015 |
| | | JP 6106899 B2 | 05 April 2017 |
| | | EP 2892904 A1 | 15 July 2015 |
| | | EP 2892904 A4 | 06 April 2016 |
| | | EP 2892904 B1 | 17 October 2018 |
| CN 116283997 A | 23 June 2023 | None | |
| CN 105326793 A | 17 February 2016 | None | |
| WO 2021222045 A1 | 04 November 2021 | EP 4142715 A1 | 08 March 2023 |
| | | CA 3181336 A1 | 04 November 2021 |
| | | JP 2023523295 A | 02 June 2023 |
| CN 101374843 A | 25 February 2009 | SV 2008002963 A | 18 January 2010 |
| | | LT 1966214 T | 10 February 2017 |
| | | RS 55630 B1 | 30 June 2017 |
| | | WO 2007075567 A1 | 05 July 2007 |
| | | NO 20083013 L | 19 September 2008 |
| | | NO 340958 B1 | 31 July 2017 |
| | | PT 1966214 T | 03 February 2017 |
| | | PE 20070752 A1 | 13 August 2007 |
| | | JP 2009525263 A | 09 July 2009 |
| | | JP 5292102 B2 | 18 September 2013 |
| | | US 2009098181 A1 | 16 April 2009 |
| | | US 8173654 B2 | 08 May 2012 |
| | | IL 192108 A0 | 29 December 2008 |
| | | IL 192108 A | 27 February 2014 |
| | | UA 98297 C2 | 10 May 2012 |
| | | HRP 20170103 T1 | 24 March 2017 |
| | | MA 30084 B1 | 01 December 2008 |
| | | US 2007203136 A1 | 30 August 2007 |
| | | US 8030305 B2 | 04 October 2011 |
| | | GT 200800105 A | 10 October 2008 |
| | | ES 2612377 T3 | 16 May 2017 |
| | | ZA 200806277 B | 29 September 2010 |
| | | NI 200800179 A | 06 January 2016 |
| | | MX 2008008277 A | 04 March 2009 |
| | | PE 20110008 A1 | 31 January 2011 |
| | | CY 1118846 T1 | 10 January 2018 |
| | | TW 200801007 A | 01 January 2008 |
| | | TWI 399378 B | 21 June 2013 |
| | | AP 200804502 A0 | 30 June 2008 |
| | | AP 2008004502 A0 | 31 October 2015 |
| | | TNSN 08278 A1 | 30 October 2009 |
| | | MY 159523 A | 13 January 2017 |
| | | DK 1966214 T3 | 13 February 2017 |
| | | CR 10170 A | 24 November 2008 |
| | | UY 30041 A1 | 31 May 2007 |
| | | PL 1966214 T3 | 28 April 2017 |

Form PCT/ISA/210 (patent family annex) (July 2022)

**INTERNATIONAL SEARCH REPORT**
**Information on patent family members**

International application No.
**PCT/CN2023/105714**

| Patent document cited in search report | Publication date (day/month/year) | Patent family member(s) | | | Publication date (day/month/year) |
|---|---|---|---|---|---|
| | | ME | 02736 | B | 20 October 2017 |
| | | AR | 058703 | A1 | 20 February 2008 |
| | | CA | 2634721 | A1 | 05 July 2007 |
| | | CA | 2634721 | C | 17 June 2014 |
| | | BRPI | 0620292 | A2 | 08 November 2011 |
| | | BRPI | 0620292 | B1 | 24 August 2021 |
| | | NZ | 568807 | A | 27 May 2011 |
| | | HN | 2008000931 | A | 22 October 2013 |
| | | EP | 1966214 | A1 | 10 September 2008 |
| | | EP | 1966214 | B1 | 02 November 2016 |
| | | EP | 1966214 | B9 | 13 September 2017 |
| | | ECSP | 088573 | A | 30 July 2008 |
| | | AP | 200804502 | D0 | 30 June 2008 |
| | | HUE | 030390 | T2 | 29 May 2017 |
| | | AU | 2006331912 | A1 | 05 July 2007 |
| | | AU | 2006331912 | B2 | 30 August 2012 |
| | | KR | 20080085154 | A | 23 September 2008 |
| | | KR | 101412675 | B1 | 03 July 2014 |
| | | EA | 200870085 | A1 | 30 December 2008 |
| | | EA | 015754 | B1 | 30 December 2011 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 103122000 A

### Non-patent literature cited in the description

- Remington, The Science and Practice of Pharmacy. Williams & Wilkins PA, 2000